# EUROPEAN PATENT SPECIFICATION

(11) **EP 2 057 466 B1**
(45) Date of publication and mention of the grant of the patent: **23.10.2013**
(21) Application number: 07836873.5
(22) Date of filing: 15.08.2007
(51) Int. Cl.: G01N 33/53, A61K 35/30, A61K 39/395, A61K 35/24, C07K 14/00, C07K 16/00

(54) **METHODS AND COMPOSITIONS FOR TREATMENT AND DIAGNOSIS OF AUTOIMMUNE ENCEPHALITIS OR EPILEPSY**
VERFAHREN UND ZUSAMMENSETZUNGEN ZUR BEHANDLUNG UND DIAGNOSE VON AUTOIMMUNER ENZEPHALITIS ODER EPILEPSIE
MÉTHODES ET COMPOSITIONS POUR LE TRAITEMENT ET LE DIAGNOSTIC DE L'ENCÉPHALITE AUTO-IMMUNE OU DE L'ÉPILEPSIE

(30) Priority: 15.08.2006 US 837624 P
(43) Date of publication of application: 13.05.2009
(73) Proprietor: The Trustees of the University of Pennsylvania, Philadelphia, PA 19104-6283 (US); CHILDREN'S HOSPITAL OF PHILADELPHIA, Philadelphia, PA 19104 (US)
(72) Inventor: DALMAU, Josep, Philadelphia, PA 19107 (US); ROSENFELD, Myrna, Philadelphia, PA 19107 (US); LYNCH, David R., Bleubell, PA 19422 (US)
(74) Representative: Dawson, Elizabeth Ann
(86) International application number: PCT/US2007/018092
(87) International publication number: WO 2008/021408

(56) References cited:
- WO-A1-2006/081188
- TAKAHASHI YUKITOSHI; MORI HISASHI; MISHINA MASAYOSHI; WATANABE MASAHIKO; KONDO NAOMI; SHIMOMURA JIRO; KUBOTA YUKO; MATSUDA KAZUMI;: "Autoantibodies and cell-mediated autoimmunity to NMDA-type GluRepsilon2 in patients with Rasmussen's encephalitis and chronic progressive epilepsia partialis continua" EPILEPSIA, RAVEN PRESS LTD, NEW YORK, US, vol. 46, no. s5, 29 June 2005 (2005-06-29) , pages 152-158, XP009122774 ISSN: 0013-9580 [retrieved on 2005-06-29]
- GANOR; GOLDBERG-STERN Y; LERMAN-SAGIE H; TEICHBERG T; LEVITE V I; M: "Autoimmune epilepsy: Distinct subpopulations of epilepsy patients harbor serum autoantibodies to either glutamate/AMPA receptor GluR3, glutamate/NMDA receptor subunit NR2A or double-stranded DNA" EPILEPSY RESEARCH, ELSEVIER SCIENCE PUBLISHERS, AMSTERDAM, NL, vol. 65, no. 1-2, 1 June 2005 (2005-06-01) , pages 11-22, XP005035497 ISSN: 0920-1211
- DALMAU JOSEP; TUEZUEN ERDEM; WU HAI-YAN; MASJUAN JAIME; ROSSI JEFFREY E; VOLOSCHIN ALFREDO; BAEHRING JOACHIM M; SHIMAZAKI HARUO; K: "Paraneoplastic anti-N-methyl-D-aspartate receptor encephalitis associated with ovarian teratoma" ANNALS OF NEUROLOGY, JOHN WILEY AND SONS, BOSTON, US, vol. 61, no. 1, 1 January 2007 (2007-01-01), pages 25-36, XP009122766 ISSN: 0364-5134 [retrieved on 2007-01-29]
- IIZUKA ET AL.: 'Anti-NMDA receptor encephalitis in Japan' NEUROLOGY vol. 70, 2004, pages 504 - 511, XP008104248
- MOCHIZUKI ET AL.: 'Acute limbic encephalitis: A new entity?' NEUROSCI. LETT. vol. 394, 2006, pages 5 - 8, XP025023440
- TUZUIN ET AL.: 'Adenylate kinase 5 autoimmunity in treatment refractory limbic encephalitis' J. NEUROIMMUNOL. vol. 186, no. 1-2, May 2007, pages 177 - 180, XP022181769
- NASKY K.M. ET AL: 'Psychosis associated with anti-N-methyl-D-aspartate receptor antibodies' CNS SPECTR. vol. 13, no. 8, 2008, pages 699 - 703, XP008104290
- ZULIANI L. ET AL: 'Paraneoplastic limbic encephalitis associated with potassium channel antibodies: value of anti-glial nuclear antibodies in identifying the tumour' J. NEUROL. NEUROSURG. PSYCHIAT. vol. 78, 2007, pages 204 - 205, XP008104288
- KALIA ET AL.: 'NMDA receptors in clinical neurology: excitatory times ahead' LANCET NEUROL. vol. 7, 2008, pages 742 - 755, XP022851612
- YING ET AL.: 'Increased numbers of coassembled PSD-95 to NMDA-receptor subunits NR2B and NR1 in human epileptic cortical dysplasia' EPILISIA vol. 45, no. 4, 2004, pages 314 - 321, XP008104252

## Description

### FIELD OF INVENTION

This invention provides methods of diagnosing or determining a cause of an autoimmune encephalitis or an epilepsy in a subject and of diagnosing a tumor in a subject, comprising the step of testing a body fluid of the subject for an antibody to an NR subunit of the NMDA receptor.

### BACKGROUND OF THE INVENTION

Disturbances of memory, behavior, cognition, and seizures can result from immune-mediated encephalitis. One cause of autoimmune encephalitis is the paraneoplastic manifestation of a neoplasm. Until now most puraneoplastic encephalitides have been associated with antibodies to intracellular onconeuronal proteins and cytotoxic T-cells presumably against the same proteins. These disorders usually associate with malignant tumors and are poorly responsive to immunotherapies or treatment of the cancer.

In recent years a severe but often reversible encephalitis of unknown etiology that predominantly affects young women has been increasingly recognized. The disorder has received several names, including acute diffuse lymphocytic meningoencephalitis, acute reversible limbic encephalitis, acute juvenile female non-herpetic encephalitis, or juvenile acute non-herpetic encephalitis. No association has yet been made with infections, cancer, or specific autoantibodies but given that most patients develop a prodromic viral-like illness, a postinfectious immune-mediated etiology has been postulated.

The affected patients were women who developed prominent psychiatric symptoms, seizures, memory deficits, and decreased level of consciousness often requiring ventilatory support. Three salient features included the young age of the patients, the association with ovarian teratomas, and the detection of antibodies to unknown antigens predominantly expressed in the cell membrane of hippocampal neurons (also referred to as a subgroup of neuropil antigens).

A better understanding of the function of the paraneoplastic neuronal (or onconeuronal) antigens along with modelling PND in animals results in improved treatment strategies. For the clinician who currently confronts these patients, however, the best chance to affect the neurologic outcome depends on: (1) the prompt diagnosis of the disorder, (2) the early discovery and treatment of the tumor, and (3) the use of immunotherapy. Accordingly, a need Exists for a reliable method of diagnosing the presence of autoimmune encephalitis and the possibility that observed epileptic seizures are caused by (sometimes occult) tumors.

This invention provides methods of diagnosing or determining a cause of an autoimmune encephalitis or an epilepsy in a subject and of diagnosing a tumor in a subject, comprising the step of testing a body fluid of the subject for an antibody to an NR subunit of the NMDA receptor.

In one embodiment, the present invention provides a method of determining a cause of an encephalitis in a subject, comprising the step of testing a body fluid of the subject for an antibody to an NR subunit of the NMDA receptor (NMDAR), thereby determining a cause of an encephalitis in a subject. In another embodiment, the presence of an antibody to an NR subunit in the body fluid indicates that the encephalitis is of autoimmune etiology. Each possibility represents a separate embodiment of the present invention.

In another embodiment, the present invention provides a method of determining a cause of an autoimmune encephalitis in a subject, comprising the step of testing a body fluid of the subject for an antibody to an NR subunit of the NMDA receptor, thereby determining a cause of an autoimmune encephalitis in a subject. In another embodiment, the presence of the antibody indicates a presence of a tumor in the subject. In another embodiment, the tumor is a cause of the autoimmune encephalitis. Each possibility represents a separate embodiment of the present invention.

In another embodiment, the present invention provides a method of determining a cause of an epilepsy in a subject, comprising the step of testing a body fluid of the subject for an antibody to an antibody to an NR subunit of the NMDA receptor, thereby determining a cause of an epilepsy in a subject. In another embodiment, the antibody indicates a presence of a tumor in the subject. In another embodiment, the tumor is a cause of the epilepsy. Each possibility represents a separate embodiment of the present invention.

In another embodiment, the present invention provides a method of diagnosing a tumor in a subject having an encephalitis, comprising the step of testing a body fluid of the subject for an antibody to an NR subunit of the NMDA receptor, thereby diagnosing a tumor in a subject having an encephalitis. In another embodiment, the presence of the antibody indicates a presence of a tumor in the subject. Each possibility represents a separate embodiment of the present invention.

In another embodiment, the present invention provides a method of diagnosing a tumor in a subject having an epilepsy, comprising the step of testing a body fluid of the subject for an antibody to an antibody to an NR subunit of the NMDA receptor, thereby diagnosing a tumor in a subject having an epilepsy. In another embodiment, the presence of the antibody indicates a presence of a tumor in the subject. Each possibility represents a separate embodiment of the present invention.

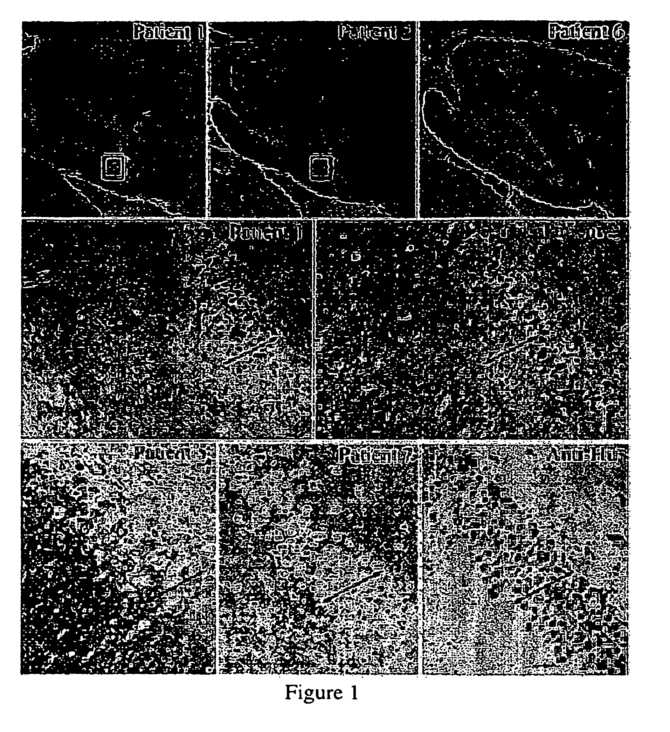

Figure 1: Hippocampal neuropil antibodies in LE of unclear etiology. Sagiital sections of rat brain reacted with sera of several LE patients. Upper row: 3 cases showing intense reactivity with the neuropil of hippocampus. Cases #2 and 6 competed for the same epitopes (not shown); case #2 had an additional antibody against a subset of neurons in the inner part of the dentate gyrus (shown in middle row). Middle and lower rows at high magnification show the reactivity of cases #1, 2, 5, 7 and a control anti-Hu. Compare the intracellular reactivity of case #7 and anti-Hu with the predominant neuropil reactivity of cases #1, 2 and 5 that spare the nuclei and cytoplasm of neurons (except for #2 that labels a subset of cells). Only case #1 had VGKC antibodies. Arrows point to the dentate gyrus to allow comparison between panels. (Upper row x5; middle and low rows x200).

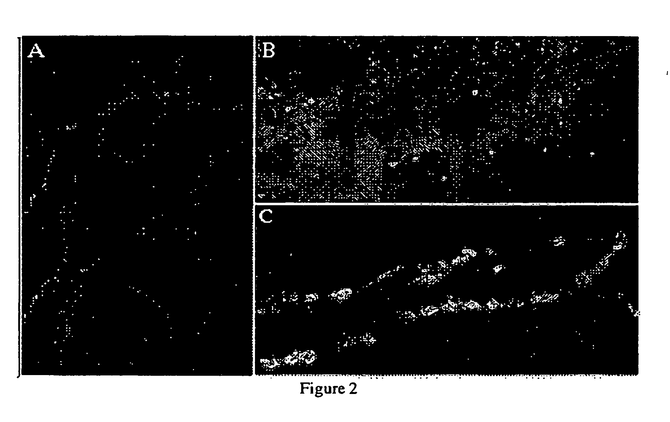

Figure 2. Immuno-labeling of neurons with novel antibodies of a patient with LE. Cultures of rat hippocampal neurons: (A) incubated with patients' CSF, (B) co-labeled with synaptophysin or (C) with spinophilin. Patient's antibodies (red in all panels in original) show intense reactivity with the cell membrane, with limited colocalization with spinophilin (yellow in C in original) and less with synaptophysin (B). (x800 oil).

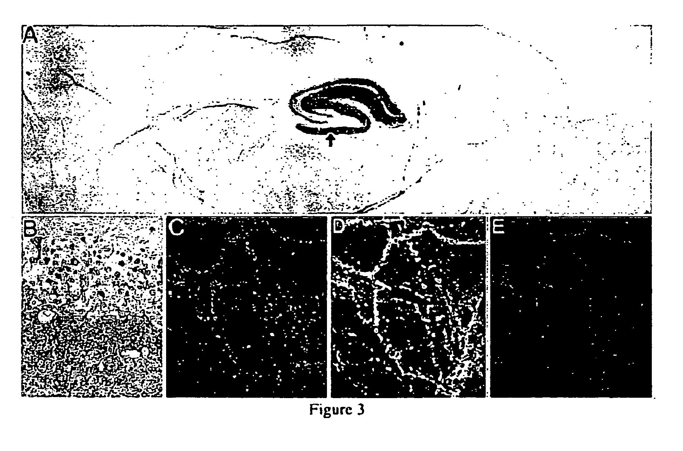

Figure 3. Reactivity of patients' antibodies with hippocampus and forebrain, and colocalization with the NR2B subunit of the N-methyl-D-aspartate receptor (NMDAR). (A) Sagittal section of rat brain immunolabeled with a patient's cerebrospinal fluid. Note the robust reactivity with the hippocampus and milder reactivity with forebrain. The cerebellum is largely spared. (B) Higher magnification of the molecular layer of the hippocampus (arrow in A); this pattern of reactivity is identical to that previously reported in patients with paraneoplastic encephalitis and ovarian teratoma.4 (C-E) Double immunolabeling of cultures of rat hippocampal neurons using a patient's antibodies (C, green) and an antibody against NR2B of the NMDAR (E, red); note the significant colocalization of reactivities (D, yellow). These findings suggested that patients' antibodies were directed against the NMDAR. Subsequent studies demonstrated that the patient also had antibodies against NR2A (not shown), which explains, in part, the partial colocalization of reactivities. Original magnification X2.5 (A) and X400 (B), both counterstained with hematoxylin; X800 (oil lens), immunofluorescence (C-E).

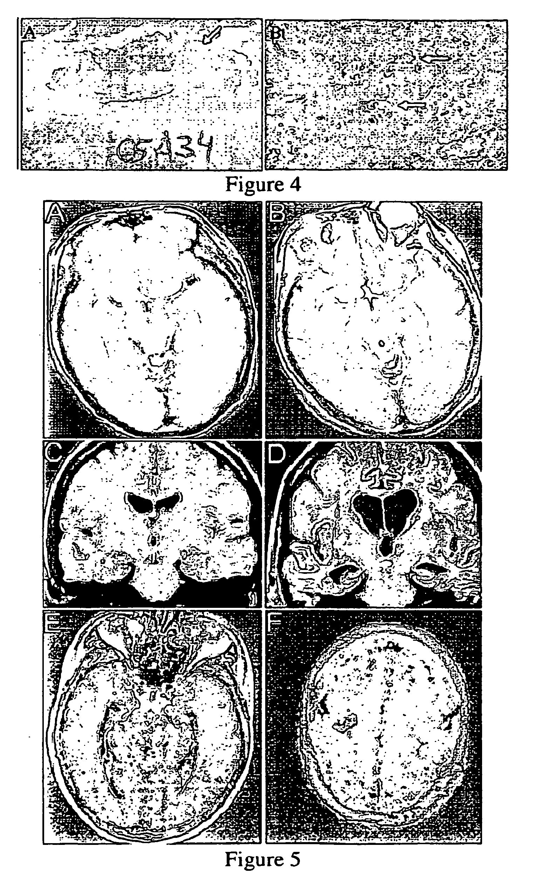

Figure 4. Ovarian teratoma from a patient with LE. (A) Macroscopic view shows presence of fat and hairs (arrow). (B) Microscopic region showing normal-appearing nervous tissue (arrows point to neurons). x200 H&

Figure 5: Brain magnetic resonance imaging (MRI) findings in three patients. (A, B) MRI of Patient I at symptom presentation (A) and after partial clinical improvement and cerebrospinal fluid normalization with immunotherapy (B); note that the clinical and MRI improvement started to occur before tumor resection. (C, D) MRI of Patient 2 at symptom presentation (C) and 4 months later (D); this patient developed rapidly progressive neurological deterioration that did not respond to immunotherapy. The autopsy demonstrated that the ovarian cyst was a mature teratoma of the ovary. (E, F) MRI of Patient 3 at symptom presentation; note the mild fluid-attenuated inversion recovery hyperintensity in medial temporal lobes and right frontal cortex. After immunotherapy and tumor resection, the MRI was normal (not shown).

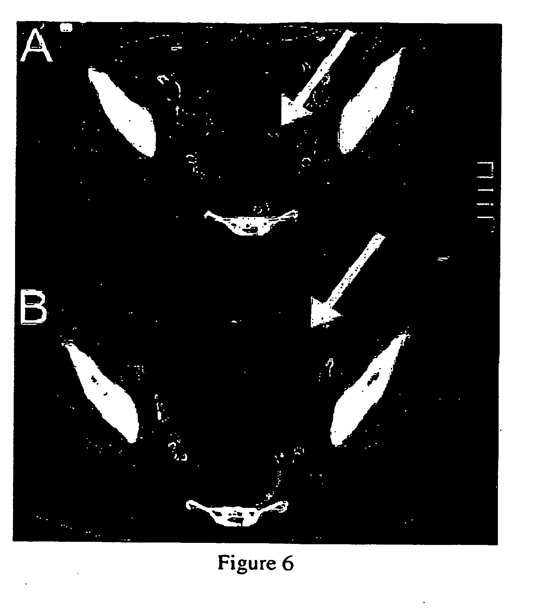

Figure 6: Interval increase of tumor size during encephalitis. (A) Computed tomography of Patient 1 shows a 5cm cystic ovarian lesion (arrow) that doubled in size over 2 months (B). The lesion was not initially removed because of the poor clinical condition of the patient and benign appearance of the ovarian mass. After partial clinical improvement with immunotherapy, the mass was removed (immature teratoma).

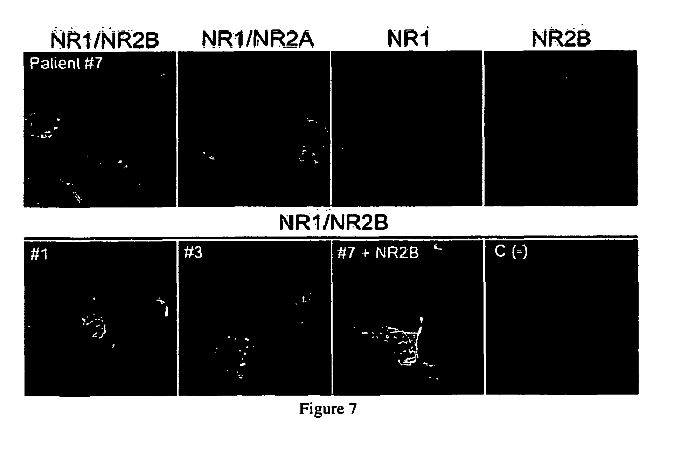

Figure 7: Patients' antibodies react with heteromers of NR2B and NR2A subunits of the N-methyl-D-aspartate receptor (NMDAR). HEK293 cells expressing heteromers (NR1/NR2B or NR1/NR2A) or transfected with single subunits NR1 or NR2B of the NMDAR were incubated with patients' serum or cerebrospinal fluid (CSF). (top row) Panels demonstrate that the CSF of Patient 7 reacts with cells expressing heteromers (functional receptors) of NR1/NR2B and NR1/NR2A, but not with cells transfected with single subunits (NR1; NR2B). Cells transfected with single NR2A or plasmid without insert were not reactive with patient's antibodies (not shown). (bottom row) Panels demonstrate the reactivity of the CSF of Patients 1 and 3 with NR1/NR2B. In the third panel (#7 + NR2B), the cells were coincubated with CSF of Patient 7 and an antibody specific for NR2B, showing colocalization of reactivities. The fourth panel (C (-)) corresponds to the CSF of an individual without paraneoplastic encephalitis (negative control). Original magnification 0 800, immunofluorescence; nuclei of cells demonstrated with 4 diamidino-2-phenylindole (DAPI), except "#7 + NR2B," in which no DAPI was used.

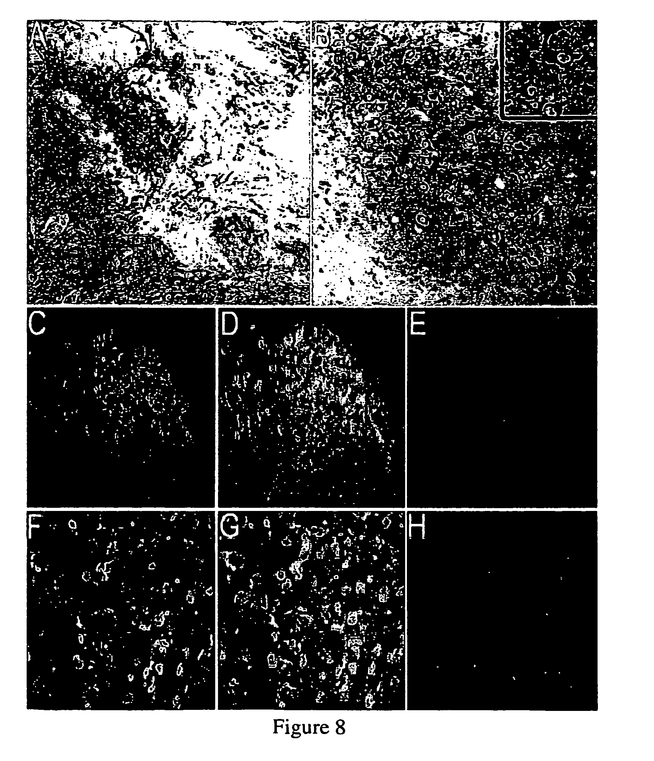

Figure 8: Patients' antibodies react with NR2 subunits of the N-methyl-D-aspartate receptors (NMDARs) expressed by nervous tissue present in the tumor. (A, B) Panels correspond to the ovarian teratoma of Patients 3 and 5 immunolabeled with MAP2 (brown staining), a marker specific for neurons and dendritic processes. Note the intense reactivity with neuronal-like cells and a network of cell processes that are better developed in A. (B, inset) Some immature neuronal cells at higher magnification. (C-E) Panels correspond to the tumor of Patient 3 immunolabeled with the patient's antibodies (C, green) and a specific antibody for NR2B (E, red). Note that there is colocalization of reactivities (D, yellow), indicating that the patient's antibodies react with NR2B expressed in the tumor (similar findings were observed with NR2A, not shown). (F-H) Panels correspond to the tumor of Patient 5 immunolabeled with the patient's antibodies (F, green) and a specific antibody for NR2B (H, red). There is also colocalization of reactivities (G, yellow), indicating that the patient's antibodies recognize NR2B expressed in the tumor (similar findings were observed with NR2A, not shown). Original magnification X200, counterstained with hematoxylin (A, B); X400, immunofluorescence (C-H).

### DETAILED DESCRIPTION OF THE INVENTION

In one embodiment, the present invention provides a method of determining a cause of an encephalitis in a subject, comprising the step of testing a body fluid of the subject for an antibody to an NR subunit of the NMDA receptor, thereby determining a cause of an encephalitis in a subject. In another embodiment, the presence of an antibody to an NR subunit in the body fluid indicates that the encephalitis is of autoimmune etiology. Each possibility represents a separate embodiment of the present invention. In one embodiment, the term "subunit" is interchangeable with the term "heteromer".

In one embodiment, the methods and compositions provided herein facilitate the recognition of a severe form of autoimmune encephalitis that is often responsive to treatment. In another embodiment, the methods and compositions described herein emphasize the idea that autoimmunity can affect behavior, and particularly that antibodies to heteromers containing the NR2B and NR2A subunits of the NMDAR may alter emotion, in one embodiment, or memory, consciousness or their compbination in other independent embodiments.

In another embodiment, the present invention provides a method of determining a cause of an autoimmune encephalitis in a subject, comprising the step of testing a body fluid of the subject for an antibody to an NR subunit of the NMDA receptor, thereby determining a cause of an autoimmune encephalitis in a subject. In another embodiment, the presence of the antibody indicates a presence of a tumor in the subject. In another embodiment, the tumor is a cause of the autoimmune encephalitis. Each possibility represents a separate embodiment of the present invention.

In another embodiment, the present invention provides a method of diagnosing a paraneoplastic autoimmune encephalitis in a subject, comprising the step of testing a body fluid of the subject for an antibody to an NR subunit of the NMDA receptor, thereby diagnosing a paraneoplastic autoimmune encephalitis in a subject.

The biological sample used in the methods described herein is a body fluid that is tested by methods of the present invention is, in another embodiment, a cerebro-spinal fluid (CSF). In another embodiment, the body fluid is plasma. In another embodiment, the body fluid is any other type of fluid known in the art. Each possibility represents a separate embodiment of the present invention. In another embodiment, the biological sample is amniotic fluids, blood, sera, saliva, or their combination in another embodiment.

The autoimmune encephalitis of methods and compositions of the present invention is, in another embodiment, an autoimmune encephalitis. In another embodiment, the autoimmune encephalitis is a paraneoplastic encephalitis. In another embodiment, the autoimmune encephalitis is a non-paraneoplastic encephalitis. In another embodiment, the autoimmune encephalitis is a paraneoplastic autoimmune encephalitis. In another embodiment, the autoimmune encephalitis is a non-paraneoplastic, autoimmune encephalitis. In another embodiment, the autoimmune encephalitis is any other type of autoimmune encephalitis known in the art. Each possibility represents a separate embodiment of the present invention.

In one embodiment, the frequency of paraneoplastic anti-NMDAR encephalitis, diagnosed by the methods described herein, is unknown. In another embodiment paraneoplastic anti-NMDAR encephalitis is frequently unrecognized. This may be due to several features that make this disorder unique among paraneoplastic encephalitis, including in one embodiment, involvement of relatively young women between the 2^{nd} and 5^{th} decades, or, in another embodiment, the unusual presentation with prominent psychiatric manifestations, or in another embodiment, normal or atypical MRI findings, which in 75% of cases consist of mild, transient T2 or FLAIR abnormalities outside the medial temporal lobes, with cortical enhancement in certain embodiments, or in yet another embodiment, the benign appearance of the ovarian tumors. In one embodiment, any of the subjects presenting the symptoms described hereinabove are diagnosed using the methods described herein.

Anti-NMDAR encephalitis is different from other types of paraneoplastic encephalitis in several ways: it results in a highly characteristic syndrome; usually affects young women; is treatment-responsive; and associates with tumors that can be benign. Another difference shown here is that despite the presence of the tumor, the immune response is not maintained. This brings into consideration a contributory role of the prodromal "viral-like" disorder, which by itself or in combination with a teratoma sets off or enhances the autoimmune response. In one embodiment, the methods provided herein are used to differentiate anti-NMDAR encephalitis from other types of paraneoplastic encephalitis.

In another embodiment, the autoimmune encephalitis of methods and compositions of the present invention comprises a limbic encephalitis. In another embodiment, the autoimmune encephalitis is a limbic encephalitis. In another embodiment, the autoimmune encephalitis is associated with a limbic dysfunction. In another embodiment, the autoimmune encephalitis is not associated with a limbic dysfunction. Each possibility represents a separate embodiment of the present invention.

In one embodiment, Limbic encephalitis causes impressive deficits that are characteristically dominated by rapid and severe loss of short-term memory. In another embodiment, patients show subacute encephalitis of later adult life, mainly affecting the limbic areas with evidence of cancer in one embodiment. In one embodiment, the term "limbic encephalitis" refers to a subject exhibiting severe short-term memory loss and dementia in association with bronchial carcinoma.

In another embodiment, the autoimmune encephalitis of methods and compositions of the present invention is associated with seizures. In another embodiment, the autoimmune encephalitis is associated with a dienoephalic syndrome. In another embodiment, the autoimmune encephalitis is associated with a psychiatric symptom. In another embodiment, the autoimmune encephalitis is associated with an abnormality in cognition. In another embodiment, the autoimmune encephalitis is associated with an abnormality in behavior.

In another embodiment, the autoimmune encephalitis is associated with amnesia. In another embodiment, the autoimmune encephalitis is associated with a memory deficit. In another embodiment, the autoimmune encephalitis is associated with memory problems. In another embodiment, the autoimmune encephalitis is associated with a hypokinetic syndrome.

In another embodiment, the autoimmune encephalitis is associated with a movement disorder. In another embodiment, the autoimmune encephalitis is associated with abnormal movements. In another embodiment, the movement disorder is Stiff Man/Person Syndrome. In another embodiment, the movement disorder is any other movement disorder known in the art. Each possibility represents a separate embodiment of the present invention.

In another embodiment, the autoimmune encephalitis is associated with a decreased level ofconsciousness. In another embodiment, the autoimmune encephalitis is associated with hypoventilation.

In another embodiment, the autoimmune encephalitis is associated with, dysfunction of any part of the brain or spinal cord. In another embodiment, the autoimmune encephalitis is associated with a combination of any of the above symptoms or disorders. Each type of encephalitis represents a separate embodiment of the present invention.

In another embodiment, the autoimmune encephalitis is associated with a tumor. In another embodiment, the tumor is an ovarian teratoma. In another embodiment, the tumor is a thymic tumor.

In another embodiment, the tumor is a testicular tumor. In another embodiment, the cancer associated with the encephalitis is a cervical cancer tumor. In another embodiment, the cancer is a head and neck cancer tumor. In another embodiment, the cancer is a breast cancer tumor. In another embodiment, the cancer is an ano-genital cancer tumor.

In another embodiment, the cancer is a melanoma. In another embodiment, the cancer is a sarcoma. In another embodiment, the cancer is a carcinoma. In another embodiment, the cancer is a lymphoma. In another embodiment, the cancer is a leukemia. In another embodiment, the cancer is mesothelioma. In another embodiment, the cancer is a glioma. In another embodiment, the cancer is a germ cell tumor. In another embodiment, the cancer is a choriocarcinoma. Each possibility represents a separate embodiment of the present invention.

In another embodiment, the cancer is pancreatic cancer. In another embodiment, the cancer is ovarian cancer. In another embodiment, the cancer is gastric cancer. In another embodiment, the cancer is a carcinomatous lesion of the pancreas. In another embodiment, the cancer is pulmonary adenocarcinoma. In another embodiment, the cancer is colorectal adenocarcinoma. In another embodiment, the cancer is pulmonary squamous adenocarcinoma. In another embodiment, the cancer is gastric adenocarcinoma. In another embodiment, the cancer is an ovarian surface epithelial neoplasm (e.g. a benign, proliferative or malignant variety thereof). In another embodiment, the cancer is an oral squamous cell carcinoma. In another embodiment, the cancer is non small-cell lung carcinoma. In another embodiment, the cancer is an endometrial carcinoma. In another embodiment, the cancer is a bladder cancer. In another embodiment, the cancer is a head and neck cancer. In another embodiment, the cancer is a prostate carcinoma.

In another embodiment, the cancer is an acute myelogenous leukemia (AML). In another embodiment, the cancer is a myelodysplastic syndrome (MDS). In another embodiment, the cancer is a non-small cell lung cancer (NSCLC). In another embodiment, the cancer is a Wilms' tumor. In another embodiment, the cancer is a leukemia. In another embodiment, the cancer is a lymphoma. In another embodiment, the cancer is a desmoplastic small round cell tumor. In another embodiment, the cancer is a mesothelioma (e.g. malignant mesothelioma). In another embodiment, the cancer is a gastric cancer. In another embodiment, the cancer is a colon cancer. In another embodiment, the cancer is a lung cancer. In another embodiment, the cancer is a breast cancer. In another embodiment, the cancer is a germ cell tumor. In another embodiment, the cancer is an ovarian cancer. In another embodiment, the cancer is a uterine cancer. In another embodiment, the cancer is a thyroid cancer. In another embodiment, the cancer is a hepatocellular carcinoma. In another embodiment, the cancer is a thyroid cancer. In another embodiment, the cancer is a liver cancer. In another embodiment, the cancer is a renal cancer. In another embodiment, the cancer is a kaposis. In another embodiment, the cancer is a sarcoma. In another embodiment, the cancer is another carcinoma or sarcoma.

In another embodiment, the tumor is any other type of tumor known in the art. Each possibility represents a separate embodiment of the present invention.

In another embodiment, the present invention provides a method of determining a cause of an epilepsy in a subject, comprising the step of testing a body fluid of the subject for an antibody to an antibody to an NR subunit of the NMDA receptor, wherein the antibody indicates a presence of a tumor, wherein a tumor is a cause of the epilepsy.

The epilepsy of methods and compositions of the present invention is, in another embodiment, an idiopathic epilepsy. In another embodiment, the epilepsy responds to IgG-depleting therapy. In another embodiment, the epilepsy is associated with partial seizures. In another embodiment, the epilepsy is associated with simple partial seizures. In another embodiment, the epilepsy is associated with complex partial seizures. In another embodiment. the epilepsy is associated with generalized seizures. In another embodiment, the epilepsy is associated with absence (petit mal) seizures. In another embodiment, the epilepsy is associated with myoclonic seizures. In another embodiment, the epilepsy is associated with tonic-clonic (grand mal) seizures.

In another embodiment, the epilepsy is associated with West syndrome. In another embodiment, the epilepsy is associated with Lennox-Gastaut syndrome. In another embodiment, the epilepsy is associated with any other syndrome known in the art.

In another embodiment the epilepsy is of no known cause. In another embodiment the epilepsy is any other type of epilepsy known in the art. Each type of epilepsy represents a separate embodiment of the present invention.

"Cause of" an autoimmune encephalitis, epilepsy, etc, refers, in another embodiment, to a primary cause of the disorder. In another embodiment, the term refers to a contributing cause of the disorder. In another embodiment, the term refers to an indirect causation. In another embodiment, the term refers to causation via an immune response induced by the tumor. In another embodiment, the term refers to a significant cause of the disorder. Each possibility represents a separate embodiment of the present invention.

In another embodiment, the present invention provides a method of diagnosing a tumor in a subject having an encephalitis, comprising the step of testing a body fluid of the subject for an antibody to an NR subunit of the NMDA receptor, thereby diagnosing a tumor in a subject having an encephalitis. In another embodiment, the presence of the antibody indicates a presence of a tumor in the subject. Each possibility represents a separate embodiment of the present invention.

In another embodiment, the present invention provides a method of detecting a tumor in a subject having an encephalitis, comprising the step of testing a body fluid of the subject for an antibody to an NR subunit of the NMDA receptor, thereby detecting a tumor in a subject having an encephalitis. In another embodiment, the presence of the antibody indicates a presence of a tumor in the subject. Each possibility represents a separate embodiment of the present invention.

In one embodiment, the present invention provides a method of diagnosing a tumor in a subject having an epilepsy, comprising the step of testing a body fluid of the subject for an antibody to an antibody to an NR subunit of the NMDA receptor, thereby diagnosing a tumor in a subject having an epilepsy. In another embodiment, the presence of the antibody indicates a presence of a tumor in the subject. Each possibility represents a separate embodiment of the present invention.

In one embodiment, the present invention provides a method of detecting a tumor in a subject having an epilepsy, comprising the step of testing a body fluid of the subject for an antibody to an antibody to an NR subunit of the NMDA (N-methyl D-aspartate) receptor, thereby detecting a tumor in a subject having an epilepsy. In another embodiment, the presence of the antibody indicates a presence of a tumor in the subject. Each possibility represents a separate embodiment of the present invention.

In one embodiment, surprisingly, all patients that have antibodies to NMDARs containing the NR2B, and at a lesser degree the NR2A subunit. In another embodiment, NMDARs are formed from heteromers of NR1 (which bind glycine) and NR2 subunits (which bind glutamate). Both subunits are required to create a functional receptor that likely contains two NR1 and two NR2 subunits. There are four NR2 subunits (NR2A-D) which have 50-70% sequence identity in the extracellular domain (NR2B is 70% identical to NR2A, and 55-58% identical to NR2C and NR2D). These NR2 subunits are coded by four different genes and show developmental and regional variability. NR2B is expressed at high levels prenatally and declines postnatally. During its decline, expression levels of NR2A and NR2C increase in certain embodiments.

In adults, NR2A is found in most brain regions, NR2B in the hippocampus and forebrain, NR2C in cerebellum, and NR2D in limited subsets of neurons. NR1 is ubiquitously distributed in the brain. With maturity many NR1/NR2B receptors become largely extrasynaptic in hippocampal neurons and NR1/NR2A/NR2B becomes the major synaptic receptors in the hippocampus and forebrain. Thus, in one embodiment, the predominant reactivity of the subjects' antibodies with hippocampus and forebrain correlates with the distribution of heteromers containing NR2B. In one embodiment, the antibodies readily access cells surface epitopes of live neurons, and only react with HEK293 cells expressing functional receptors (heteromers of NR1/NR2B or NR1/NR2A).

In one embodiment, reactivity is identified when each subunit is expressed individually (even though NR1 in particular assembles in stable but inactive homomeric receptors in HEK293 cells) or in anotherembodiment, with immunoblots of cells expressing the functional receptor. These findings suggest that the main epitopes are in the extracellular domain of NR2B and NR2A subunits, and are likely conformational. In another embodiment, subjects harbor antibodies to functional heteromers of NR2C and NR2D (consistent with the 55-58% homology between these subunits and NR2B). In one embodiment, no increase of cerebellar immunolabeling is observed using the methods described herein in sera or CSF from these subjects, indicating that in certain embodiments, these antibodies occur at very low titers.

The NR subunit of methods and compositions of the present invention is, in another embodiment, a NR2B subunit. In another embodiment, the NR subunit is a GRIN2B (glutamate receptor, ionotropic, N-methyl D-aspartate 2B subunit. In another embodiment, the NR2B subunit is part of an NMDA heteromer. In another embodiment, the heteromer is a functional heteromer. In another embodiment, the NR subunit is a NR2A subunit. In another embodiment, the NR subunit is a GRIN2A (glutamate receptor, ionotropic, N-methyl D-aspartate 2A) subunit. In another embodiment, the NR2A subunit is part of an NMDA heteromer. In another embodiment, the heteromer is a functional heteromer In another embodiment, the NR subunit is a NR2C subunit. In another embodiment, the NR subunit is a GRIN2C (glutamate receptor, ionotropic, N-methyl D-aspartate 2C subunit). In another embodiment, the NR2C subunit is part of an NMDA heteromer.. In another embodiment, the heteromer is a functional heteromer In another embodiment, the NR subunit is any other NR subunit known in the art. Each possibility represents a separate embodiment of the present invention.

The NR2B subunit has, in another embodiment, the sequence:

In another embodiment, the NR2B subunit is a homologue of SEQ ID No: 1. In another embodiment, the NR2B subunit is a variant of SEQ ID No: 1. In another embodiment, the NR2B subunit is an isomer of SEQ ID No: 1. In another embodiment, the NR2B subunit is a fragment of SEQ ID No: 1. In another embodiment, the NR2B subunit comprises SEQ ID No: 1. Each possibility represents a separate embodiment of the present invention.

In another embodiment, the NR2B subunit is encoded by a nucleotide sequence having the sequence:

In another embodiment, the NR2B subunit is encoded by a nucleotide molecule that a homologue of SEQ ID No: 2. In another embodiment, the nucleotide molecule is a variant of SEQ ID No: 2. In another embodiment, the nucleotide molecule is an isomer of SEQ ID No: 2. In another embodiment, the nucleotide molecule is a fragment of SEQ ID No: 2. In another embodiment, the nucleotide molecule comprises SEQ ID No: 2. Each possibility represents a separate embodiment of the present invention.

In other embodiments, the NR2B subunit has a sequence set forth in 1 of the following GenBank entries: U28861, U28862, U28758, U90278, U11287, U88963, BC113618, BC113620, and AB208850. In other embodiments, the NR2B subunit has a sequence that is a variant of 1 of the above sequences. In other embodiments, the NR2B subunit has a sequence that is a homologue of 1 of the above sequences. In other embodiments, the NR2B subunit has a sequence that is an isomer of 1 of the above sequences. In other embodiments, the NR2B subunit has a sequence that is a fragment of 1 of the above sequences. In other embodiments, the NR2B subunit has a sequence that comprises 1 of the above sequences. In another embodiment, the NR2B subunit is encoded by a sequence set forth in 1 of the above entries. In another embodiment, the NR2B subunit is encoded by a homologue of a sequence set forth in 1 of the above entries. In another embodiment, the NR2B subunit is encoded by a variant of a sequence set forth in 1 of the above entries. In another embodiment, the NR2B subunit is encoded by an isomer of a sequence set forth in 1 of the above entries. Each sequence, and each possibility, represents a separate embodiment of the present invention.

In another embodiment, the NR2A subunit of methods and compositions of the present invention has the sequence:

In another embodiment, the NR2A subunit is a homologue of SEQ ID No: 3. In another embodiment, the NR2A subunit is a variant of SEQ ID No: 3. In another embodiment, the NR2A subunit is an isomer of SEQ ID No: 3. In another embodiment, the NR2A subunit is a fragment of SEQ ID No: 3. In another embodiment, the NR2A subunit comprises SEQ ID No: 3. Each possibility represents a separate embodiment of the present invention.

In another embodiment, the NR2A subunit is encoded by a nucleotide sequence having the sequence:

In another embodiment, the NR2A subunit is encoded by a nucleotide molecule that a homologue of SEQ ID No: 4. In another embodiment, the nucleotide molecule is a variant of SEQ ID No: 4. In another embodiment, the nucleotide molecule is an isomer of SEQ ID No: 4. In another embodiment, the nucleotide molecule is a fragment of SEQ ID No: 4. In another embodiment, the nucleotide molecule comprises SEQ ID No: 4. Each possibility represents a separate embodiment of the present invention.

In other embodiments, the NR2A subunit has a sequence set forth in I of the following GenBank entries: U09002, U90277, BC117131, AB209695, and NP_000824. In other embodiments, the NR2A subunit has a sequence that is a variant of 1 of the above sequences. In other embodiments, the NR2A subunit has a sequence that is a homologue of 1 of the above sequences. In other embodiments, the NR2A subunit has a sequence that is an isomer of 1 of the above sequences. In other embodiments, the NR2A subunit has a sequence that is a fragment of 1 of the above sequences. In other embodiments, the NR2A subunit has a sequence that comprises 1 of the above sequences. In another embodiment, the NR2A subunit is encoded by a sequence set forth in 1 of the above entries. In another embodiment, the NR2A subunit is encoded by a homologue of a sequence set forth in I of the above entries. In another embodiment, the NR2A subunit is encoded by a variant of a sequence set forth in 1 of the above entries. In another embodiment, the NR2A subunit is encoded by an isomer of a sequence set forth in 1 of the above entries. Each sequence, and each possibility, represents a separate embodiment of the present invention.

In another embodiment, the NR2C subunit of methods and compositions of the present invention has the sequence:

In another embodiment, the NR2C subunit is a homologue of SEQ ID No: 5. In another embodiment, the NR2C subunit is a variant of SEQ ID No: 5. In another embodiment, the NR2C subunit is an isomer of SEQ ID No: 5. In another embodiment, the NR2C subunit is a fragment of SEQ ID No: 5. In another embodiment, the NR2C subunit comprises SEQ ID No: 5. Each possibility represents a separate embodiment of the present invention.

In another embodiment, the NR2C subunit is encoded by a nucleotide sequence having the sequence:

In another embodiment, the NR2C subunit is encoded by a nucleotide molecule that a homologue of SEQ ID No: 6. In another embodiment, the nucleotide molecule is a variant of SEQ ID No: 6. In another embodiment, the nucleotide molecule is an isomer of SEQ ID No: 6. In another embodiment, the nucleotide molecule is a fragment of SEQ ID No: 6. In another embodiment, the nucleotide molecule comprises SEQ ID No: 6. Each possibility represents a separate embodiment of the present invention.

In other embodiments, the NR2C subunit has a sequence set forth in 1 of the following GenBank entries: L76224, U77782, BC031077, BC059384, and AB208799. In other embodiments, the NR2C subunit has a sequence that is a variant of 1 of the above sequences. In other embodiments, the NR2C subunit has a sequence that is a homologue of 1 of the above sequences. In other embodiments, the NR2C subunit has a sequence that is an isomer of 1 of the above sequences. In other embodiments, the NR2C subunit has a sequence that is a fragment of 1 of the above sequences. In other embodiments, the NR2C subunit has a sequence that comprises 1 of the above sequences. In another embodiment, the NR2C subunit is encoded by a sequence set forth in 1 of the above entries. In another embodiment, the NR2C subunit is encoded by a homologue of a sequence set forth in 1 of the above entries. In another embodiment, the NR2C subunit is encoded by a variant of a sequence set forth in 1 of the above entries. In another embodiment, the NR2C subunit is encoded by an isomer of a sequence set forth in 1 of the above entries. Each sequence, and each possibility, represents a separate embodiment of the present invention.

The epitope recognized by an antibody detected by a method of the present invention is, in another embodiment, a conformational epitope. In another embodiment, the epitope is a linear epitope. In another embodiment, the epitope is any other type of epitope known in the art. Each possibility represents a separate embodiment of the present invention.

The discovery of NR2B-related antibodies in the serum and CSF of all patients provides in one embodiment, a diagnostic test for the paraneoplastic anti-NMDAR encephalitis disorder, and provides a novel immune-mediated mechanism of NMDAR dysfunction. In one embodiment, critical roles of NMDARs include synaptic transmission and remodeling, or dendritic sprouting, and hippocampal long-term potentiation in other embodiments in addition to one paradigm of memory formation and learning. In another embodiment, NMDARs are also the major mediator of excitotoxicity, and their dysfunction has been associated with schizophrenia, epilepsy, and several types of dementia. Drugs interacting with NMDARs may result in paranoia in one embodiment, or hallucinations and dyskinesias in certain embodiments, all frequent symptoms in subjects diagnosed using the methods described herein.

In one embodiment, ectopic expression of NR2 subunits by nervous tissue contained in the teratomas contributes to break immune tolerance. In another embodiment, a combination of factors such as an adjuvant effect of the prodromal viral-like illness that occur in most subjects, and a genetic predisposition in certain embodiments, play additional roles in the initiation of the immune response tested for using the diagnosis methods described herein.

A possible pathogenic role of NR2 antibodies in paraneoplastic anti-NMDAR encephalitis is shown by the correlation between patients' symptoms and antibody Liters in one embodiment or, in another embodiment, by the demonstration of deposits of IgG in the hippocampus and amygdala of a patient's autopsy, in a pattern that showed striking resemblance to the rat brain immunolabeling by patients' antibodies (See Figure 6). While in other paraneoplastic encephalitis (i.e., Hu, Ma2) the perivascular and interstitial infiltrates of T-cells are prominent,^{34,35} the encephalitis with NR2 antibodies associates with extensive microglial proliferation, variable neuronal degeneration, and rare inflammatory infiltrates (Figure 7). In these patients and a previously reported case (#11)⁸ the abnormalities predominated in the hippocampi and amygdala, and at a lesser degree in other areas of the neuraxis.

In another embodiment, the subject has exhibits antibodies that react with SIZN1 (Smad-Interacting Zinc finger protein expressed in the Nervous system).

In another embodiment, the subject has exhibits antibodies that react with a VGKC antigen.

In another embodiment, the subject exhibits antibodies that react with an extracellular neuronal antigen. In another embodiment, the subject exhibits antibodies that react with an antigen exposed on the cell surface of a neuron. As provided herein (Example 9), patients with antibodies to extracellular antigens exhibit, under the conditions utilized herein, enhanced responsiveness to immune therapy.

In another embodiment, a method of the present invention utilizes, detects, or tests for a target antigen (other than a NR2B subunit) identified by a method disclosed herein. In another embodiment, the target antigen is identified by a library screening technique. In another embodiment, the target antigen is identified by cDNA library screening. In another embodiment, the target antigen is identified by reactivity with cultured neurons. In another embodiment, the target antigen is identified by a protocol set forth in Example 8. Each possibility represents a separate embodiment of the present invention.

Methods for testing a reactivity of a serum against neuronal antigens are well known in the art. In another embodiment, one of the methods enumerated in the Examples herein is utilized. In another embodiment, neuronal tissue is fixed with PFA. In another embodiment, any other method know in the art is utilized. Each possibility represents a separate embodiment of the present invention.

Methods and kits for detection of antibodies are well known in the art, and are described, for example, in Ances BM et al (Treatment-responsive limbic encephalitis identified by neuropil antibodies: MRI and PET correlates. Brain 2005;128:1764-1777) and Vitaliani et al (Paraneoplastic encephalitis, psychiatric symptoms, and hypoventilation in ovarian teratoma. Ann Neurol 2005; 58:594-604.).

Methods for diagnosing limbic encephalitis (LE) are well known in the art. In another embodiment, patients with LE develop subacute confusion, irritability, depression, sleep disturbances, seizures, short-term memory loss, and/or dementia. In another embodiment, the pathological substrate of LE is an inflammatory disorder that predominantly involves the limbic system (hippocampi, amygdala, and cingulate gyrus). In another embodiment, biopsy and autopsy studies demonstrate interstitial and perivascular infiltrates ofT cells, and less frequently B cells, along with microglial activation, neuronal degeneration, and/or gliosis. In another embodiment, inflammatory infiltrates are found in areas distant from the limbic system. In another embodiment, the infiltrates remain mild and clinically silent. In another embodiment, the infiltrates become prominent and develop into a disorder called encephalomyelitis. Additional methods of diagnosing LE are described, for example, in Gultekin SH et al (Paraneoplastic limbic encephalitis: neurological symptoms, immunological findings and tumour association in 50 patients. Brain 2000;123:1481-1494). Each possibility represents a separate embodiment of the present invention.

In another embodiment, an antigen of the present invention is homologous to a peptide disclosed herein. The terms "homology," "homologous," etc, when in reference to any protein or peptide, refer, in one embodiment, to a percentage of amino acid residues in the candidate sequence that are identical with the residues of a corresponding native polypeptide, after aligning the sequences and introducing gaps, if necessary, to achieve the maximum percent homology, and not considering any conservative substitutions as part of the sequence identity. Methods and computer programs for the alignment are well known in the art.

Homology is, in another embodiment, determined by computer algorithm for sequence alignment, by methods well described in the art. For example, computer algorithm analysis of nucleic acid sequence homology can include the utilization of any number of software packages available, such as, for example, the BLAST, DOMAIN, BEAUTY (BLAST Enhanced Alignment Utility), GENPEPT and TREMBL packages.

In another embodiment, "homology" refers to identity to a sequence selected from SEQ ID No: 1-6 of greater than 70%. In another embodiment, "homology" refers to identity to a sequence selected from SEQ ID No: 1-6 of greater than 72%. In another embodiment, "homology" refers to identity to one of SEQ ID No: 1-6 of greater than 75%. In another embodiment, "horology" refers to identity to a sequence selected from SEQ ID No: 1-6 of greater than 78%. In another embodiment, "homology" refers to identity to one of SEQ ID No: 1-6 of greater than 80%. In another embodiment, "homology" refers to identity to one of SEQ ID No: 1-6 of greater than 82%. In another embodiment, "homology" refers to identity to a sequence selected from SEQ ID No: 1-6 of greater than 83%. In another embodiment, "homology" refers to identity to one of SEQ ID No: 1-6 of greater than 85%. In another embodiment, "homology" refers to identity to one of SEQ ID No: 1-6 of greater than 87%. In another embodiment. "homology" refers to identity to a sequence selected from SEQ ID No: 1-6 of greater than 88%. In another embodiment, "homology" refers to identity to one of SEQ ID No: 1-6 of greater than 90%. In another embodiment, "homology" refers to identity to one of SEQ ID No: 1-6 of greater than 92%. In another embodiment, "homology" refers to identity to a sequence selected from SEQ ID No: 1-6 of greater than 93%. In another embodiment, "homology" refers to identity to one of SEQ ID No: 1-6 of greater than 95%. In another embodiment, "homology" refers to identity to a sequence selected from SEQ ID No: 1-6 of greater than 96%. In another embodiment, "homology" refers to identity to one of SEQ ID No: 1-6 of greater than 97%. In another embodiment, "homology" refers to identity to one of SEQ ID No: 1-6 of greater than 98%. In another embodiment, "homology" refers to identity to one of SEQ ID No: 1-6 of greater than 99%. In another embodiment, "homology" refers to identity to one of SEQ ID No: 1-6 of 100%. Each possibility represents a separate embodiment of the present invention.

In another embodiment, homology is determined via determination of candidate sequence hybridization, methods of which are well described in the art (See, for example, "Nucleic Acid Hybridization" Hames, B. D., and Higgins S. J., Eds. (1985); Sambrook et al., 2001, Molecular Cloning, A Laboratory Manual, Cold Spring Harbor Press, N.Y.; and Ausubel et al., 1989, Current Protocols in Molecular Biology. Green Publishing Associates and Wiley Interscience, N.Y). In other embodiments, methods of hybridization are carried out under moderate to stringent conditions, to the complement of a DNA encoding a native caspase peptide. Hybridization conditions being, for example, overnight incubation at 42 °C in a solution comprising: 10-20 % formamide, 5 X SSC (150 mM NaCl, 15 mM trisodium citrate), 50 mM sodium phosphate (pH 7. 6), 5 X Denhardt's solution, 10 % dextran sulfate, and 20 µg/ml denatured, sheared salmon sperm DNA.

Protein and/or peptide homology for any AA sequence listed herein is determined, in another embodiment, by methods well described in the art, including immunoblot analysis, or via computer algorithm analysis of AA sequences, utilizing any of a number of software packages available, via established methods. Some of these packages include the FASTA, BLAST, MPsrch or Scanps packages, and, in another embodiment, employ the use of the Smith and Waterman algorithm, and/or global/local or BLOCKS alignments for analysis, for example. Each method of determining homology represents a separate embodiment of the present invention.

In another embodiment of the present invention, "nucleic acids" or "nucleotide" refers to a string of at least two base-sugar-phosphate combinations. The term includes, in one embodiment, DNA and RNA. "Nucleotides" refers, in one embodiment, to the monomeric units of nucleic acid polymers. RNA is, in one embodiment, in the form of a tRNA (transfer RNA), snRNA (small nuclear RNA), rRNA (ribosomal RNA), mRNA (messenger RNA), anti-sense RNA, small inhibitory RNA (siRNA), micro RNA (miRNA) and ribozymes. The use of siRNA and miRNA has been described (Caudy AA et al, Genes & Devel 16: 2491-96 and references cited therein). DNA can be, in other embodiments, in form of plasmid DNA, viral DNA, linear DNA, or chromosomal DNA or derivatives of these groups. In addition, these forms of DNA and RNA can be single, double, triple, or quadruple stranded. The term also includes, in another embodiment, artificial nucleic acids that contain other types of backbones but the same bases. In one embodiment, the artificial nucleic acid is a PNA (peptide nucleic acid). PNA contain peptide backbones and nucleotide bases and are able to bind, in one embodiment, to both DNA and RNA molecules. In another embodiment, the nucleotide is oxetane modified. In another embodiment, the nucleotide is modified by replacement of one or more phosphodiester bonds with a phosphorothioate bond. In another embodiment, the artificial nucleic acid contains any other variant of the phosphate backbone of native nucleic acids known in the art. The use of phosphothiorate nucleic acids and PNA are known to those skilled in the art, and are described in, for example, Neilsen PE, Curr Opin Struct Biol 9:353-57; and Raz NK et al Biochem Biophys Res Commun. 297:1075-84. The production and use of nucleic acids is known to those skilled in art and is described, for example, in Molecular Cloning, (2001), Sambrook and Russell, eds. and Methods in Enzymology: Methods for molecular cloning in eukaryotic cells (2003) Purchio and G. C. Fareed. Each nucleic acid derivative represents a separate embodiment of the present invention.

### EXPERIMENTAL DETAILS SECTION

### EXAMPLE 1: Paraneoplastic anti-NMDA Receptor Encephalitis Associated with Ovarian Teratoma MATERIALS AND EXPERIMENTAL METHODS

### Patient selection

Patients include 12 women with paraneoplastic encephalitis associated with teratomas. The 6 most recently identified patients and neuropathological findings (2 cases) are described in detail in the supplementary files; the clinical features of the other 6 patients have been previously reported. Frozen serum or cerebrospinal fluid was available from all 12 patients. Tissues for immunological studies included tumors from 5 patients (1 frozen tissue, 4 embedded in paraffin), and brain obtained at autopsy of 1 patient and 2 neurologically normal individuals. Sera or CSF of 200 individuals, including blood donors and patients with diverse paraneoplastic and non-paraneoplastic encephalitis served as controls. Studies were approved by the University of Pennsylvania Institutional Review Board.

### Animal tissue, antibodies, and IgG biotinylation

Wistar rats were sacrificed omitting perfusion with saline or fixatives; the brain was removed, immersed in 4% paraformaldehyde (PFA) at 4°C for 24 hours, cryoprotected with 40% sucrose, sagittally sectioned and snap frozen in isopentane chilled with liquid nitrogen. The following antibodies were used at the indicated dilutions: chicken anti-MAP2 (Covance, Princeton, NJ; 1:20,000); rabbit anti-NR 1 (amino acids 1-20) and rabbit anti-NR2A (amino acids 1265-1464) (both from Upstate biotechnology, Lake Placid, NY, both at 1:50); rabbit anti-NR2B (251 amino acid sequence from N-terminal portion of NMDAR; Zymed, San Francisco, CA, 1:50); CD3, CD19 and CD68 (DakoCytomation, Carpinteria, CA, all 1:100).

To avoid reactivity with endogenous IgG, all immunohistochemical studies with tumor tissue utilized IgG purified from patients' sera and labeled with biotin.

### Immunohistochemistry

Paraffin-embedded tissue was deparaffinized and the antigens retrieved, as reported.¹⁰ Seven micron-thick frozen (or 4 micron-thick paraffin) tissue sections were serially incubated with 0.3% H₂O₂ for 20 minutes, 10% goat serum for 1 hour, and patient's serum (1:250), CSF (1:10) or biotinylated IgG (0.2 mg/ml), or the indicated purchased antibodies overnight at 4°C. After using the appropriate secondary antibodies (all 1:2000), reactivities were developed with the avidin-biotin-peroxidase method. The secondary antibody was omitted when patients' biotinylated IgG was used. Normal human serum and biotinylated IgG, and normal goat serum served as controls. Double immunolabeling with patients' IgG, MAP2, and NR2 antibodies was performed using the appropriate Alexa Fluor secondary antibodies diluted 1:2000 (Molecular Probes, OR); results were photographed under a fluorescence microscope using Zeiss Axiovision software.

### Immunocytochemistry

Rat hippocampal neuronal cultures were prepared as reported.¹¹ Live neurons grown on coverslips were exposed for 1 hour at 37°C to the patients' or control serum (final dilution 1:1000) orCSF (1:100). After removing the media and extensive washing, neurons were fixed with 4% PFA, and single or double immunolabeling was performed, as indicated above. The reactivity of commercial antibodies to NR1 and NR2 subunits was best seen with cell permeabilization (0.1% Triton) after PFA.

HEK293 cells were transfected with plasmids containing rodent NR1, NR2A, or NR2B subunits of the NMDAR (>90% homologous to the human subunits in the extracellular domains) or plasmids without insert (control), as reported. In other experiments cells were co-transfected with NR1 and NR2A (or NR1 and NR2B) in equimolar ratios. Cells were grown for 24 hours after transfection before assessment. All cells were routinely grown in the presence of NMDAR antagonists (500 µM ketamine) to prevent cell death after transfeciion.¹⁵ Transfected cells were then incubated with patients' serum (1:400) or CSF (1:10) overnight at 4°C, and the appropriate Alexa Fluor secondary antibodies, as above.

### RESULTS

### Neurological findings

The median age of the patients was 27 years (range 14-44 years). In 11 patients the neurologic symptoms preceded the diagnosis of the teratoma by 3 weeks-4 months (median 2 months) and in 1 patient occurred after an ovarian cyst had been radiologically detected one month earlier. Ten patients had a viral-like prodromic syndrome with hyperthermia; (7 cases) and frequent headache (6 cases) (Table 1).

Three of the 12 patients presented with short-term memory loss, followed by psychiatric symptoms or confusion and decreased level of consciousness (Table 1). The other 9 patients presented with an acute psychiatric syndrome, including personality and behavioral change, agitation or paranoid thoughts. Overall, 6 patients were initially evaluated by psychiatrists and 5 admitted to psychiatric units. Eleven patients developed generalized or partial complex seizures. After controlling the seizures, 10 patients required mechanical ventilation due to the decreased level of consciousness and hypoventilation in 9 cases, and a pulmonary embolism in 1; the median time that patients were on mechanical ventilation was 12 weeks (2-16 weeks). During that time multiple EEGs showed diffuse general slowing in 7 patients, and generalized slowing with occasional epileptic activity in the other 3.

During the course of the disease, 8 patients developed episodes of hyperthermia alternating in 1 case with hypothermia (Table 1). 7 patients developed abnormal movements that included one or more of the following: choreoathetosis, myoclonic and ballistic movements, dystonic movements, dyskinesias in face and arms, rhythmic contractions of the abdominal wall, opisthotonos-like postures, and catatonic-like episodes. 5 patients had signs of autonomic instability, including episodes of mydriasis, tachycardia, tachypnea, diaphoresis, or hypertension which in 1 case alternated with hypotension. 4 patients developed transient sleep dysfunction while recovering from the encephalitis.

**Table 1: Clinical features**

| **Case Sex/ age** | **Teratoma: histology, side, size** | **Time to tumor diagnosis** | **Prodrome** | **Presenting symptoms** | **Other symptoms and findings during the course of the disorder** |
|---|---|---|---|---|---|
| # 1 F/30 | immature, right ovary, 10 cm | 2 months | headache, hyperthermia | STMD, decreased level of consciousness, "epilepsia partialis continua" for 2 weeks. Sedation, MV, PEG | Restlessness, involuntary movements hyperthermia |
| # 2 F/35 | mature, left ovary, 3.5 cm | 4 months (autopsy) | headache, nausea, no fever; received acetaminop hen | STMD, generalized tonic-clonic seizure followed by refractory status epilepticus. Sedation, MV, and PEG | Partial motor seizures in left lower extremity; dystonic movements; hyperthermia. |
| # 3 F/25 | mature, left ovary, 6cm | 6 weeks | hyperthermia | STMD, panic attacks, confusion, hallucinations (admitted to psychiatric center). Subsequently, partial complex seizures, TPN, PEG. | Episodes of staring; minimally reactive to stimuli. Catatonic-like episodes. Autonomic instability: tachycardia, high blood pressure. |
| # 4 F/17 | immature, left ovary, 7 cm | 4 weeks | hyperthermia | Bizarre behavior, disorganized thinking, restless, wandering aimlessly, hallucinations, catatonic-like episodes (2 admissions in psychiatric centers). Subsequently, status epilepticus. Sedation, MV. | Incoherent speech; episodes of staring, catatonia, oculogyric crisis, choreoathetoid movements. Autonomic instability: hyperthermia; mydriasis during episodes of restlessness and agitation. |
| # 5 F/32 | mature, right ovary, 6cm | 2 months | NA | Acute development of personality change, abnormal behavior, confused, agitated, wandering off (admitted to psychiatry). Generalized TC seizures, partial motor seizures, severe general encephalopathy, PEG. | Unable to speak and follow-commands for 2 months; episodes of "facial twitches". |
| # 6 F/24 | mature, right ovary, 1.5 cm | 3 months (autopsy) | headache, hypenhermia, nausea, vomiting, diarrhea | Paranoid thoughts, auditory hallucinations, agitation (admitted to psychiatric). Subsequently, generalized seizure; sedation, MV, PEG. Remained with decreased level of consciousness and ventilator dependant. | Myoclonic and dyskinetic movements in arms and face. Autonomic instability: alternating hypo-hyperthermia; hypo-hypertension. During awake periods, tachypnea; during sleep periods, problem triggering ventilator. |

| **Previously reported patients** | | | | | |
|---|---|---|---|---|---|
| # 7⁶ F/19 | immature, right ovary, 22 cm | 4 months | headache, hyperthermia | Acute personality change, aggressive behavior (seen at a psychiatric center). Progressive confusion, decreased level of consciousness. General and partial seizures, refractory status epilepticus. Sedation, MV, PEG. | Episodes of "chewing, grimacing"; myoclonic and ballistic movements with limbs; rhythmic contractions of abdominal wall. Dystonic movements of the trunk and limbs; opisthotonos-like posture; insomnia-hypersomnia. |
| # 8⁴ F/26 | left ovarian dermoid cyst by CT*, 1.6 cm | 3 weeks | anorexia and insomnia (3 weeks) | Psychiatric syndrome, generalized seizures, MV, PEG | Incomprehensible speech, decreased of level of consciousness, STMD, hyperthermia |
| # 9⁴ F/40 | mature, left ovary, 6.0 cm | 3 weeks | NA | Secondary generalized seizures, psychiatric syndrome, MV, PEG | Decreased of level of consciousness, STMD |
| # 10^{4.8} F/14 | immature, left ovary, 1.9 cm | 2 months | hyperthermia, headache, rhinorrhea | Psychiatric syndrome (hallucinations, extreme panic), generalized seizures, MV, PEG | Incomprehensible speech, choreoathetotic movements, hypersomnia, hyperthermia, autonomic instability |
| # 11^{4.7} F/28 | 1^{st}: mature, right ovary, 14 cm; | 1 month after tumor | cough,no fever; received antibiotics | 1^{st} episode: psychiatric syndrome (delusional thinking, personality change), auditory hallucinations, STMD, dysphagia, horizontal nystagmus, vertical gaze paresis, MV | 1^{st} episode: hypersomnia, comatose, flaccid paraplegia. |
| | 2^{nd}: "benign", left, 2 cm; | | | 2^{nd} episode: dysarthria; 3^{rd} episode: diplopia, facial numbness, dysphagia, ataxia. | |
| | 3^{rd}: "benign", left, 1.7 cm | | | | |
| # 12⁵ F/44 | mature, mediastinu m, 6.5 cm | 3 weeks | hyperthermia, headache | Admission to psychiatry for acute agitation, personality change, memory loss, generalized tonic-clonic seizures, MV for PE, PEG | Word finding difficulty, mild right hemiparesis, episodes of diaphoresis, hypersomnia, headache. |

### Ancillary tests

All patients underwent several brain MRIs at the early stage of the disorder (Table 2): 3 had bilateral media temporal lobe FLAIR hyperintensities (1 with right parietal cortex involvement) (Figure 5); 5 had small a punctuate areas of FLAIR or T2 hyperintensity in the frontal or parietal cortex (2 with cerebellar involvement) and subtle enhancement of overlying meninges; 1 had transient T2 hyperintensity in the medulla and spinal cord, and had normal or non-specific findings.

CSF lymphocytic pleocytosis was identified in all patients (9-219 cells/□1, median 24), and 4 also had elevated protein concentration (56-129 mg/dL, median 67); the glucose concentration was normal in all instances (Table 2). Oligoclonal bands were identified in 3 of 6 patients examined. All patients had extensive serum and CSF diagnostic tests with negative or normal results for viral, bacterial, fungal infections, collagen-vascular autoimmune disorders, thyroid autoimmunity, and comprehensive panels of paraneoplastic and VGKC antibodies.

**Table 2: Diagnostic tests, treatment, and outcome**

| **Case** | **MRI at presentation** | **CSF** | **Chronologic list of treatments** | **Initial improvement** | **Outcome** |
|---|---|---|---|---|---|
| | | | **(immuno-therapy and tumor)** | | **(duration follow-up)** |
| # 1 | FLAIR and T2 hyperintensity in medial temporal lobes | 40 WBC, protein 67 | corticosteroids, plasma exchange, IVIg | Partial clinical and MRI improvement presurgery. Further improvement 4 weeks after surgery | Back to work as an internal medicine resident; normal MRI (12 months). |
| | | | (tumor removal) | | |
| # 2 | FLAIR and T2 hyperintensity in medial temporal lobes | 189 WBC, protein 68, | corticosteroids, plasma exchange, IVIg, cyclophosphamide | No surgery (did not improve) | Follow-up MRIs: severe atrophy, mainly in temporal lobes. Died 4 months after symptom presentation. |
| | | (+) OGB | | | |
| # 3 | Initial MRI normal. Subsequent MRI: FLAIR hyperintensity in medial temporal lobes and right frontal cortex | 15 WBC, | corticosteroids, | Three days post-surgery | Normal exam; normal MRI (12 months). |
| | | protein normal | (tumor removal), | | |
| | | (-) OGB | plasma exchange | | |
| # 4 | Single punctuate FLAIR abnormality in the right frontal lobe. Two subsequent MRIs: normal | 26 WBC, | plasma exchange, IVIg, | 12 days post-surgery, and 3 days postcorticosteroids and bolus of cyclophosphamide: striking improvement, able to communicate and be extubated | Back to high school with good grades. Normal exam; normal MRI (7 months). |
| | | protein normal | (tumor removal), corticosteroids and cyclophosphamide | | |
| # 5 | Two MRIs normal | 20 WBC, | corticosteroids, | 2 days after of plasma exchange (1 day before surgery). Three days after surgery: able to sit, talk, eat. | Normal exam; normal MRI (7 months). |
| | | protein 56 | plasma exchange (2 days) (tumor removal), | | |
| | | | plasma exchange (3days) | | |
| # 6 | T2 hyperintensity in sulci of parietal hemispheres; mild enhancement of overlying meninges. | 219 WBC, protein 129, | corticosteroids | No surgery (did not improve) | Died 3 months after symptom presentation. |
| | | (+) OGB | | | |

| **Previously reported patients** | | | | | |
|---|---|---|---|---|---|
| # 7⁶ | Small FLAIR abnormality in cerebellum. Mild enhancement of meninges of cerebral sulci. | 12 WBC, | corticosteroids, IVIg, | 4 weeks after surgery | Alternating insomnia-hypersomnia for 7 months. |
| | | protein normal | (tumor removal) and chemotherapy | | Total recovery of motor function, but MMSE 24/30 (mild STMD). MRI: fronto-temporal atrophy (16 months). |
| # 8⁴ | FLAIR/ T2 hyperintensities in cerebral cortex and cerebellum; mild cortical cerebellar enhancement. | 49 WBC, | corticosteroids | ∼ 7 weeks | Full recovery; normal MRI (24 months). |
| | | protein 67 | | | |
| # 9⁴ | FLAIR abnormalities involving the cingulum and gray matter of the frontal lobes | 9 WBC, | tumor removal | ∼ 16 weeks | Residual cognitive dysfunction and memory problem, MMSE 28/30 (6 years). |
| | | protein normal | | | |
| # 10^{4.8} | Three MRIs normal | 115 WBC, | tumor removal, plasma exchange, costicosteroids, IVIg | transferred to a chronic care facility with MV (no significant improvement) | Unexpected death after mild improvement, ∼6 months after symptom presentation. |
| | | protein 92 | | | |
| | | (+) OGB | | | |
| # 11^{4.7} | T2 hyperintensity in the dorsal aspect of the medulla and 3 similar areas in the spinal cord. | 1^{st} episode: | each episode: tumor removal, IVIg, corticosteroids | 1^{st} episode: ∼8 weeks | 1^{st} episode: recovered memory and cognitive function; residual mild truncal dyscsthesias (6 months) |
| | | 23 WBC, | | 2^{nd} episode: 6 weeks | |
| | | protein 61 | | 3^{rd} episode: 2 weeks | |
| | | 2^{nd}, 3^{rd} episodes: normal. | | | 2^{nd}, 3^{rd} episodes: complete recovery (22 months) |
| | | (-) OGB | | | |
| # 12⁵ | Nonspecific, scattered T2 foci of hyperintensity in frontal lobes. No contrast enhancement | 15 WBC, protein 18, (-) OGB | tumor removal | ∼1 week post-surgery | Full recovery; MRI unchanged, 4 years. |

### Associated tumors

CT of the chest, abdomen and pelvis revealed an ovarian mass in 10 patients, a tumor in the anterior mediastinum in 1, and no evidence of tumor in 1 (the tumorwas demonstrated at autopsy). The radiological size of the tumors ranged from 1.5 to 22 cm (median largest diameter 6.5 cm) (Table 1). Two patients (cases #1 and 7) had significant tumor growth during the encephalitic process (Figure 6A and B). Only one patient had elevated levels of CEA, CA125 and α-fetoprotein. One patient had a history of a resected contralateral teratoma (without accompanying encephalitis), and another patient had 3 episodes of neurologic symptoms, each heralding a new or recurrent mature teratoma (NR2 antibodies were measured at the last recurrence)

Nine patients had complete tumor resection and 3 did not have surgery (the tumor of 2 of these cases was studied at autopsy). Overall, pathological studies showed that 7 patients had mature teratoma (6 ovary, I mediastinum) and 4 immature teratoma. Review of slides from 8 of the ovarian teratomas demonstrated in all instances nervous tissue intermixed with tissues derived from the other germinal layers; from 5 of these tumors tissue was available for immunological studies (described below).

### Treatment and outcome

Seven patients were treated with tumor resection and immunosuppressants (1 with additional chemotherapy): 6 recovered and 1 died unexpectedly in a chronic care facility after mild improvement (Table 2). Five of the 6 patients who recovered, returned to work and the follow-up MRIs were normal; the sixth patient had partial recovery (MMSE 24/30) and developed mild fronto-temporal atrophy.

The other 5 patients were treated with surgery alone (2 cases) or immunosuppressants (3). Three patients recovered (2 returning to work; 1 partial recovery MMSE 28/30) and the other 2 died of neurologic progression (both without tumor removal). In one of these patients (case #2), corticosteroids, plasma exchange and IVIg had no effect on the symptoms and CSF pleocytosis; multiple MRIs revealed progressive atrophy, mainly in the temporal lobes and hippocampi. Seven weeks before her death, she received cyclophosphamide that resulted in normalization of the CSF but no clinical recovery. In the other deceased patient (case #6) the ovarian teratoma was discovered at autopsy; among many diagnostic tests this patient had undergone brain biopsy. The neuropathological findings of these 2 patients are described in the supplemental information.

### Patients' antibodies react with neuronal cell membrane antigens preferentially expressed in the hippocampus

The CSF and serum of all 12 patients, but not controls, showed a distinctive pattern of reactivity with the neuropil of rat hippocampus; the reactivity with other forebrain regions was less intense, and hardly visible in the cerebellum (Figure 3 A). The immunolabeling predominantly occurred with the cell membrane of neurons and was very intense in the molecular layer of the hippocampus (Figure 3 B). The addition of CSF or serum of patients, but not controls, to cultures of live rat hippocampal neurons, produced striking immunolabeling of the cell surface and dendrites (Figure 3 C). Control antibodies to intracellular antigens (i.e., HuD) produced no reactivity with live neurons, but showed intracellular reactivity with permeabilized neurons (data not shown). These findings indicate that the patients' antibodies recognize epitopes exposed on the cell surface.

Using rat brain tissue and serial dilutions of normalized total IgG in paired serum and CSF samples, 8 of 11 patients had evidence of intrathecal synthesis of antibodies (in 2 patients the antibodies were barely detectable in serum). Serum antibody titers were followed in 8 patients: all 7 patients with neurologic improvement had a decrease of titers (5 became undetectable), and 1 who died of neurologic progression had an increase of titers (from 1:200 to 1:1,600).

### The main autoantigens are functional heteromers of NMDARs

Double immunolabeling of brain and hippocampal rat neurons using patients' antibodies and diverse markers of candidate autoantigens demonstrated significant co-localization with the NR2B subunit of the NMDAR (Figure 3C-E). Because this subunit is preferentially expressed in the hippocampus and forebrain (and absent from the cerebellum) forming heteromers with NR1 or with NR1 and NR2A, we subsequently examined the reactivity of patients' antibodies with HEK293 cells expressing individual subunits (NR1, NR2A or NR2B) or a combination of subunits required for a functional receptor (NR1/NR2B or NR1/NR2A heteromers). These studies showed that all 12 patients' CSF and serum reacted with NR1/NR2 heteromers containing NR2B; sera and CSF from 8 patients also recognized heteromers containing NR1/NR2A (Figure 7). Sera did not react with cells transfected with individual subunits (NR1, NR2A or NR2B). These antibodies were not identified in an extensive group of control sera and CSF, including among others 6 patients with testicular teratomas and paraneoplastic anti-Ma2 encephalitis.

Immunoblots of membrane proteins isolated from hippocampal neurons or HEK293 cells expressing NR1/NR2B or NR1/NR2A heteromers, showed reactivity with commercially available antibodies (NR1, NR2A, NR2B) but did not react with patients' CSF or serum (data not shown). Overall, these findings indicate that patients' antibodies recognize NR1/NR2 heteromers containing the NR2B (and at a lesser degree NR2A) subunit of the NMDAR, and suggest that the epitopes are conformational.

Subsequently it was examined whether patients' tumors contained nervous tissue expressing NMDARs and whether these receptors were recognized by patients' antibodies. Five of 5 tumors examined showed matureand immature-appearing neurons along with a variably dense network of fibers expressing MAP2 (a marker of neurons and dendritic processes) (Figure 8A and B). This atypical nervous tissue had intense expression of NR2 subunits of NMDAR; co-localization of reactivities was observed when tumors were incubated with commercially available antibodies to NR2B or NR2A and patients' antibodies (Figure 8C-H).

### EXAMPLE 2: IDENTIFICATION OF IMMUNE RESPONSES IN PATIENTS WITH AUTOIMMUNE ENCEPHALITIS OF UNKNOWN ETIOLOGY

### MATERIALS AND EXPERIMENTAL METHODS

### Paraformaldehyde (PFA)-fixed tissue

Rats were anaesthesized and euthanized by decapitation without tissue perfusion; brains were removed and kept for 10 days in 4% PFA at 4 °C. Subsequently, brains were cryoprotected with 30% sucrose for 48 h, embedded in freezing medium, and snap-frozen in isopentane chilled with liquid nitrogen.

### Other tissue processing

Brains from rats perfused with 4% PFA were removed and kept in 4% PFA for 1 h, and subsequently cryoprotected and embedded in freezing medium as above. Non-perfused rat brains were removed and directly embedded in freezing medium without fixative.

### Immunoblot and immunohistochemistry

Sera (diluted 1:500) and CSF (1:10) were examined for antibodies using immunoblot avidin-biotin peroxidase assay, as reported. Immunoblots included protein extracts (100 microgram (mcg)/ml) from purified human cortical neurons, Purkinje cells and the recombinant proteins, HuD, Cdr2, Nova, Ma1, Ma2, CRMP5 and amphiphysin.

Immunohistochemistry was performed with cryostat-cut 7 mm thick sections mounted directly on slides. Non-pre-fixed tissue was incubated for 10 min with acetone or methanol-acetone at 4 °C. Subsequently, all tissue sections were serially incubated with 0.25% H₂O₂ for 20 min, 10% goat serum for 30 min, the patient's serum or CSF at the indicated dilutions in 10% goat serum overnight at 4 °C, biotinylated goat anti-human IgG (1 : 2000) for 2 h and avidin-biotin peroxidase for 1 h, and the reactivity developed with diaminobenzidine. Other primary antibodies used in consecutive tissue sections included: polyclonal rabbit antibodies to VGKCs Kv1.1, Kv1.2 and Kv1.6 (dilution 1:50; all from Sigma, St Louis, MO); a monoclonal antibody to Kv1.2 (1:50; Upstate Laboratories, Lake Placid, NY); a polyclonal antibody to synaptophysin (1:1000, Sigma); a monoclonal antibody to spinophilin (1:50; Upstate Laboratories); and human control serum with amphiphysin and Hu antibodies (1:500).

Intrathecal synthesis of antibodies was determined using scrum and CSF samples normalized with the same concentration of IgG and serially diluted in parallel. Patients were considered to have intrathecal synthesis of antibodies if the end dilution point of CSF showed reactivity that was no longer present in the paired serum containing the same amount of IgG.

### RESULTS

Several patients exhibiting encephalitis of unknown etiology or "possible paraneoplastic LE" were tested for anti-Ma immunity. While some patients were Ma positive, serologic testing in many of the cases was negative for all known paraneoplastic antibodies as well as other antibodies that may associate with encephalitis, memory disturbances or seizures, such as VGKC antibodies, thyroid peroxidase antibodies, glutamic-acid decarboxylase (GAD) antibodies, and antibodies associated with disorders such as lupus and Sjogren's. Since suspicion for a paraneoplastic etiology was high, cancer evaluations were carried out despite the absence of known paraneoplastic antibodies. Body CT combined with FDG-PET in 7 of the first patients studied demonstrated a tumor in 4. These findings, along with the detection of CSF abnormalities similar to those found in immune mediated disorders and the improvement of some patients with immunotherapy, led us to postulation that immune mechanisms were involved to which current screening methods were insensitive.

Several different immunohistochemical methods and tissue fixation strategies were then designed. For example, in addition to using perfused rat brain non-perfused brain fixed in paraformaldehyde (PFA) and cryoprotected with sucrose was used, and also immunolabeling of live cultures of rat neurons (from hippocampus, cortex, etc). These methods were able to visualize antibodies that were barely detectable or completely missed with other fixatives (i.e., amphiphysin, GAD or VGKC antibodies missed with acetone or methanol-acetone). Examination of the sera or CSF of the 7 patients that were antibody negative by routine screening revealed that 6 had intense reactivity predominantly concentrated in the neuropil of the hippocampus orcerebellum, involving regions enriched in dendrites and synapses and largely sparing the cytoplasm and nuclei of neurons (Figure 1). The immunohistochemical appearance of cell membrane, dendritic and synaptic staining (Figure 2A) was supported by partial co-localization with known markers, including, among others, spinophilin (dendritic spines), synaptophysin (synapses), psd95 (postsynaptic marker), and less frequently sv2 (presynaptic marker) (Figures 2B-C). Only 1/7 patients had antibodies against VGKC (confirmed by double labeling with specific Kv1.1 and Kv1.2 antibodies, and dendrotoxin radioimmunoassay). Immunocompetition studies demonstrated that 3 / 5 cases with novel antibodies to cell membrane antigens competed for the same epitopes; for example, preincubation of tissue or cells with serum of a patient abrogated the reactivity of the other patients' biotinylated IgG suggesting that they target the same auto-antigen(s).

Subsequently, additional subjects with LE were recruited. Within the category of patients with LE and novel cell membrane antibodies, a subgroup of young women with ovarian teratoma was identified. The initial cohort was 4 young women, ages 14 to 40 years, who presented with an acute severe psychotic episode that was believed to be secondary to malingering or unsubstantiated drug abuse. These patients had agitation, paranoid and delusional thinking, memory loss, seizures, progressive decrease of level of consciousness and central hypoventilation. All had MRI or FDG-PET evidence of temporal lobe involvement and CSF inflammatory abnormalities, but none had serum or CSF antibodies to Ma proteins or other well characterized antigens. Using the modified immuno-histochemical methods, it was found that the serum and/or CSF of the 4 patients contained antibodies with two unique properties: 1) the target antigens were highly enriched in the hippocampus (Figure 7), and 2) the antibodies were more readily detected in the CSF (with intrathecal synthesis) than in sera.

Immune-competition assays between patients' antibodies showed that they partially blocked the hippocampal reactivity of each other, indicating that some, but not all epitopes are common targets. The dose temporal association between presentation of the encephalitis and the diagnosis of an ovarian teratoma (median, 3 months) and the correlation in one case between three episodes of neurological relapse and tumor recurrence shows that the tumor played a role in triggering the immune response.

### EXAMPLE 3: IDENTIFICATION OF ADDITIONAL MARKERS FOR TREATMENT-RESPONSIVE AUTOIMMUNE ENCEPHALITIS

### MATERIALS AND EXPERIMENTAL METHODS

### Immunoprobing cDNA libraries:

Uni-ZAP-XR limbic and cerebellum libraries (Stratagene) are screened at a density of 5 x 10⁴ pfu/150 mm plate. Nitrocellulose filters with phage plaques are incubated with sera (pooled from 5 patients with similar antibodies, each diluted 1:1,000) or CSF (diluted 1:5) overnight at 4°C, then sequentially incubated with biotinylated goat anti-human IgG (1:2000) for 2 hours and avidin-biotin-peroxidase for 1 hour, and development under direct visual guidance with 0.05% diaminobenzidine. Clones yielding positive results are purified by several rounds of antibody screening until a yield of 100% positive plaques is obtained. Phage clones are subdoned in pBluescript using the *in vivo* excision phage rescue protocol (Stratagene) and sequenced.

### Serum Pre-absorption:

To reduce background and non-specific reactivity, the pooled (or individual) sera to be screened is preabsorbed with *E. coli* proteins. *E. coli* XL-1 blue are grown overnight in 25 ml of LB media and pelleted. The pellet is lysed in 1% Triton-X 100, and cell debris removed by centrifugation. The patients' sera are incubated with the supernatant containing the *E. coli* proteins (ratio 1 to 10) for 1 hour. Prior to screening, the mixture of sera and *E. coli* proteins is diluted with PBS so that the final dilution of each patient's serum is 1:1000.

### Screening of sera/CSF using phage plaques and immunoblot techniques:

After a clone has been retrieved, the ability of each individual serum or CSF in the pool to recognize the done is tested using a rapid screening approach. The phage clone of interest is mixed with an irrelevant (negative) phage clone at a ratio of 50/50 and plated onto agar plates yielding a lawn of phage plaques that are 50% plaques of interest and 50% irrelevant plaques. These plaques are then transferred to nitrocellulose filters that are cut into sections and incubated with each individual patient's or control sera (1:1000) or CSF (1:10), followed by the indicated biotinylated goat anti-human IgG and the avidin-biotin-peroxidase method. Positive sera strongly react with ∼50% of the plaques; negative sera show no or mild background reactivity with 100% of the plaques. The juxtaposition of negative and positive plaques provides a dear visual signal that significantly reduces false positive readings. When a clone is identified by antibodies from several patients, this clone is prioritized for retrieval and further study.

### Immunoprecipitation of solubilized radiolabelled neuronal proteins or biotinylated rat brain synaptosomes.

Neuronal cells: To label cells, media is removed from neuronal cultures of 3-week-old hippocampal neurons, and cells are washed in PBS and incubated in methionine-free Neurobasal medium (Gibco-Invitrogen) supplemented with B27 serum free su pplement and 50 U/ml penicillin-streptomycin with 1% bovine serum albumin for 60 minutes. Cells are washed and incubated with methionine-free DMEM with 0.5 mCi/ml ³⁵[S]-L-methionine for 60 minutes at 37°C, placed on ice, and washed 2 times in ice-cold PBS. This is followed by solubilization by either of the following methods. The choice of method is initially based on the presumed antigen location using double immuno-labeling techniques with well characterized proteins (pre-synaptic, post-synaptic, dendritic spines, etc).

Method A: Used for antigens that are neuronal membrane proteins. It employs Triton X-114 which undergoes cloud point precipitation at 30°C, yielding a detergent phase into which membrane and other hydrophobic proteins preferentially partition. Cells are solubilized in 50 mM Tris-HCl pH 7.4,100 mM NaCl, 0.5% Triton-X 114 (w/v), with protease inhibitors (1 mM PMSF and CLAP buffer at 1 to 1000). Cloud point precipitation is carried out by placing the mixture over a bed of 6% (w/v) sucrose and incubating for 3 minutes at 30°C or until the solution becomes cloudy followed by centrifugation. The detergent phase is removed from under the sucrose and resuspended in 50 mM Tris, pH 7.4,100 mM NaCl, 1 mM PMSF to original volume.

Method B: Used for antigens that are freely soluble neuronal proteins and some membrane proteins. Cells are lysed in 0.5 ml of 50 mM Tris-HCl pH 7.4,100 mM NaCl, 05% Triton-X 100 (w/v), and 1 mM PMSF.

Synaptosomes: Used for antigens that are enriched in synaptosomes. The region of interest is dissected from rat brain and homogenizing in 0.32M sucrose in 5 mM Tris-HCl, pH 7.4 with 2 mM EDTA. After centrifugation (750 g) the supernatant is retrieved and centrifuged at 17,000 g. The pellet is lysed in Tris-HCl pH 82 with 1 mM EDTA and homogenized, followed by centrifugation at 100,000 g. The pellet is resuspended in 0.1 M KCl, 10 mM Tris HCl, pH 7.4, 1% Triton X-100 and 2 mM EDTA and centrifuged again at 100,000 g. The synaptosome-containing supernatant is retrieved and stored until use. All buffers contain a mixture of several protease inhibitors. To biotinylate the synaptosomes, 3 µg of biotin (as a 50 µg/µl solution in DMSO) is added for each 1µg of synaptosome protein, and incubated at 4 °C overnight, and dialysed against PBS. Biotinylated synaptosomes are incubated with primary antibody in 0.1% BSA in PBS (ratio of 5 µl patient's serum, or 50 µl CSF, to 100 µg protein).

Solubilized protein extracts are preabsorbed by incubating with 30 µl protein A/G Sepharose beads and 4 µl of normal human sera for 3 hours. The pre-absorbed extracts are retrieved and incubated with the patient's serum or CSF at a ratio of 5 µl sera (50 µl CSF) to 100 µl of extract and 25 µl of protein A/G beads overnight at 4°C, washed, resuspended in Laemmli buffer and run on an SDS-PAGE immunoblot. After protein transfer to nitrocellulose, protein is visualized by XR autoradiography or avidin-biotin-peroxidase and diaminobenzidine.

If necessary to further optimize immunoprecipitation, each of the cellular fractions (cell membrane, cytosolic, synaptosomes) is further fractionated. Prior to performing precipitation on subfractions, dot blots are used as a quick and simple screening of small amounts of material, and only positive fractions are then generated.

### Controls:

Sepharose beads are pelleted after pre-absorption with normal human sera, washed, resuspended in Laemmli and run on SDS-PAGE as a control for non-specific binding. Positive controls for localization include synaptic vesicle 2, Kvl.2 VGKC, and amphiphysin; the latter located at the cytoplasmic side of the cell membrane).

### Antigen identification:

Protein bands of interest (common bands precipitated by more than one sera in a group) are excised and subjected to matrix-assisted laser desorption ionization time of flight mass spectroscopy (MALDI-MS) using the core Protein Chemistry facility of Cancer Center at the University of Pennsylvania.

### RESULTS

cDNA library screening and immunoprecipitation (IP) with rat hippocampal proteins extracted from brain or neuronal cultures (Figure 11) are used to identify additional paraneoplastic antigens. 3-week-old hippocampal neurons, which are enriched with many of the auto-antigens that are isolated herein, are used to purify the hippocampal proteins. These antigens are predominantly expressed in rat hippocampus and cerebellum, and in the cell membrane of neurons in culture (Figures 5-6). Studies focus on antigens that are targeted by several patients' antibodies, as determined by immunohistochemical competition studies and/or immunoblot.

Novel auto-antigens are tested for association with specific sub-phenotypes of LE. Tests to identify the novel auto-antigens include immunoblot, IP, and ELISA techniques.

### EXAMPLE 4: NR2B AND NR2A HETEROMERS OF THE NMDA RECEPTOR ARE THE AUTOANTIGENS OF OVARIAN TERATOMA-ASSOCIATED ENCEPHALITIS

### MATERIALS AND EXPERIMENTAL METHODS

Serum/CSF antibodies were analyzed using neuronal cultures, as described above, and HEK293 cells expressing NR subunits of the NMDA receptor.

### RESULTS

Eleven female patients (14-40 years) developed sub-acute encephalitis characterized by prominent psychiatric symptoms, seizures, and life-threatening complications (psychiatric symptoms, amnesia, seizures, dyskinesias, and decreased level of consciousness requiring ventilatory support). All the patients exhibited antibodies that reacted with extracellular conformational epitopes of NR2B (11/11) and NR2A (7/11) subunits of the NMDA receptor. NR2B binds glutamate and forms heteromers (NR1/NR2B or NR1/NR2A/NR2B) that are preferentially expressed in adult forebrain/hippocampus. Co-expression of functional heteromers was required for antibody binding. All patients were found to have an ovarian teratoma (7 mature/4 immature); all 5 tumors out of 5 tested contained nervous tissue that strongly expressed NR2B and reacted with patients' antibodies. Thus, the pathological immune response was triggered by the tumor

Tumor resection and immunotherapy (IgG-depleting strategies) resulted in recovery of 7/8 patients, paralleled by decreased antibodies titers. 2/3 patients without tumor resection died of neurologic deterioration, as evidenced by autopsy findings.

These findings demonstrate that NMDA receptor antibodies can be used as a diagnostic test for autoimmune encephalitis. In addition, these findings show that NMDA receptor antibodies are likely to play a causative role in epilepsy and immune-mediated disorders of memory, cognition, and behavior.

### EXAMPLE 5: CONFIRMATION OF PATHOLOGICAL ROLE OF NMDA RECEPTOR ANTIBODIES

In additional experiments, the functional effects of antibodies against NMDA and/or other auto-antigens identified from previous Examples are examined in live neuronal cultures, using electrophysiology with single cell recording and voltage clamp experiments. Pathological effects provide additional information regarding the physiological role of these antibodies in causation of encephalitis, epilepsy, and other disorders.

### EXAMPLE 6: PATHOLOGICAL ROLE OF NMDA RECEPTOR ANTIBODIES IN RASMUSSEN'S ENCEPHALITIS

Rasmussen's encephalitis patients are tested for the presence of anti-NMDA antibodies. Patients testing positive for the antibodies are administered treatment for depletion of anti-NMDA antibodies.

### EXAMPLE 7: PATHOLOGICAL ROLE OF NMDA RECEPTOR ANTIBODIES IN REM-ASSOCIATED SLEEP-DISORDERS

Patients having REM-associated sleep-disorders are tested for the presence of anti-NMDA antibodies. Patients testing positive for the antibodies are administered treatment for depletion of anti-NMDA antibodies.

### SEQUENCE LISTING

<110> Trustees of the University of Pennsylvania Dalmau, Joseph
<120> METHODS AND COMPOSITIONS FOR TREATMENT AND
   DIAGNOSIS OF AUTOIMMUNE ENCEPHALITIS OR EPILEPSY
<130> P-9055-PC
<150> 60/837, 624
   <151> 2006-08-15
<160> 6
<170> PatentIn version 3.4
<210> 1
   <211> 1484
   <212> PRT
   <213> Human
<400> 1
   Met Lys Pro Arg Ala Glu Cys Cys Ser Pro Lys Phe Trp
   Leu Val Leu
   1 5 10
   15
   Ala Val Leu Ala Val Ser Gly Ser Arg Ala Arg Ser Gln
   Lys Ser Pro
   20 25
   30
   Pro Ser Ile Gly Ile Ala Val Ile Leu Val Gly Thr Ser
   Asp Glu Val
   35 40 45
   Ala Ile Lys Asp Ala His Glu Lys Asp Asp Phe His His
   Leu Ser Val
   50 55 60
   Val Pro Arg Val Glu Leu Val Ala Met Asn Glu Thr Asp
   Pro Lys Ser
   65 70 75
   80
   Ile Ile Thr Arg Ile Cys Asp Leu Met Ser Asp Arg Lys
   Ile Gln Gly
   85 90
   95
   Val Val Phe Ala Asp Asp Thr Asp Gln Glu Ala Ile Ala
   Gln Ile Leu
   100 105
   110
   Asp Phe Ile Ser Ala Gln Thr Leu Thr Pro Ile Leu Gly
   Ile His Gly
   115 120 125
   Gly Ser Ser Met Ile Met Ala Asp Lys Asp Glu Ser Ser
   Met Phe Phe
   130 135 140
   Gln Phe Gly Pro Ser Ile Glu Gln Gln Ala Ser Val Met
   Leu Asn Ile
   145 150 155
   160
   Met Glu Glu Tyr Asp Trp Tyr Ile Phe Ser Ile Val Thr
   Thr Tyr Phe
   165 170
   175
   Pro Gly Tyr Gln Asp Phe Val Asn Lys Ile Arg Ser Thr Ile Glu Asn
   180 185
   190
   Ser Phe Val Gly Trp Glu Leu Glu Glu Val Leu Leu Leu Asp Met Ser
   195 200 205
   Leu Asp Asp Gly Asp Ser Lys Ile Gln Asn Gln Leu Lys Lys Leu Gln
   210 215 220
   Ser Pro Ile Ile Leu Leu Tyr Cys Thr Lys Glu Glu Ala Thr Tyr Ile
   225 230 235
   240
   Phe Glu Val Ala Asn Ser Val Gly Leu Thr Gly Tyr Gly Tyr Thr Trp
   245 250
   255
   Ile Val Pro Ser Leu Val Ala Gly Asp Thr Asp Thr Val Pro Ala Glu
   260 265
   270
   Phe Pro Thr Gly Leu Ile Ser Val Ser Tyr Asp Glu Trp Asp Tyr Gly
   275 280 285
   Leu Pro Ala Arg Val Arg Asp Gly Ile Ala Ile Ile Thr
   Thr Ala Ala
   290 295 300
   Ser Asp Met Leu Ser Glu His Ser Phe Ile Pro Glu Pro
   Lys Ser Ser
   305 310 315
   320
   Cys Tyr Asn Thr His Glu Lys Arg Ile Tyr Gln Ser Asn
   Met Leu Asn
   325 330
   335
   Arg Tyr Leu Ile Asn Val Thr Phe Glu Gly Arg Asn Leu
   Ser Phe Ser
   340 345
   350
   Glu Asp Gly Tyr Gln Met His Pro Lys Leu Val Ile Ile
   Leu Leu Asn
   355 360 365
   Lys Glu Arg Lys Trp Glu Arg Val Gly Lys Trp Lys Asp
   Lys Ser Leu
   370 375 380
   Gln Met Lys Tyr Tyr Val Trp Pro Arg Met Cys Pro Glu
   Thr Glu Glu
   385 390 395
   400
   Gln Glu Asp Asp His Leu Ser Ile Val Thr Leu Glu Glu
   Ala Pro Phe
   405 410
   415
   Val Ile Val Glu Ser Val Asp Pro Leu Ser Gly Thr Cys
   Met Arg Asn
   420 425
   430
   Thr Val Pro Cys Gln Lys Arg Ile Val Thr Glu Asn Lys
   Thr Asp Glu
   435 440 445
   Glu Pro Gly Tyr Ile Lys Lys Cys Cys Lys Gly Phe Cys
   Ile Asp Ile
   450 455 460
   Leu Lys Lys Ile Ser Lys Ser Val Lys Phe Thr Tyr Asp
   Leu Tyr Leu
   465 470 475
   480
   Val Thr Asn Gly Lys His Gly Lys Lys Ile Asn Gly Thr
   Trp Asn Gly
   485 490
   495
   Met Ile Gly Glu Val Val Met Lys Arg Ala Tyr Met Ala
   Val Gly Ser
   500 505
   510
   Leu Thr Ile Asn Glu Glu Arg Ser Glu Val Val Asp Phe
   Ser Val Pro
   515 520 525
   Phe Ile Glu Thr Gly Ile Ser Val Met Val Ser Arg Ser
   Asn Gly Thr
   530 535 540
   Val Ser Pro Ser Ala Phe Leu Glu Pro Phe Ser Ala Asp
   Val Trp Val
   545 550 555
   560
   Met Met Phe Val Met Leu Leu Ile Val Ser Ala Val Ala
   Val Phe Val
   565 570
   575
   Phe Glu Tyr Phe Ser Pro Val Gly Tyr Asn Arg Cys Leu
   Ala Asp Gly
   580 585
   590
   Arg Glu Pro Gly Gly Pro Ser Phe Thr Ile Gly Lys Ala
   Ile Trp Leu
   595 600 605
   Leu Trp Gly Leu Val Phe Asn Asn Ser Val Pro Val Gln
   Asn Pro Lys
   610 615 620
   Gly Thr Thr Ser Lys Ile Met Val Ser Val Trp Ala Phe
   Phe Ala Val
   625 630 635
   640
   Ile Phe Leu Ala Ser Tyr Thr Ala Asn Leu Ala Ala Phe Met Ile Gln
   645 650
   655
   Glu Glu Tyr Val Asp Gln Val Ser Gly Leu Ser Asp Lys Lys Phe Gln
   660 665
   670
   Arg Pro Asn Asp Phe Ser Pro Pro Phe Arg Phe Gly Thr Val Pro Asn
   675 680 685
   Gly Ser Thr Glu Arg Asn Ile Arg Asn Asn Tyr Ala Glu Met His Ala
   690 695 700
   Tyr Met Gly Lys Phe Asn Gln Arg Gly Val Asp Asp Ala Leu Leu Ser
   705 710 715
   720
   Leu Lys Thr Gly Lys Leu Asp Ala Phe Ile Tyr Asp Ala Ala Val Leu
   725 730
   735
   Asn Tyr Met Ala Gly Arg Asp Glu Gly Cys Lys Leu Val Thr Ile Gly
   740 745
   750
   Ser Gly Lys Val Phe Ala Ser Thr Gly Tyr Gly Ile Ala
   Ile Gln Lys
   755 760 765
   Asp Ser Gly Trp Lys Arg Gln Val Asp Leu Ala Ile Leu Gln Leu Phe
   770 775 780
   Gly Asp Gly Glu Met Glu Glu Leu Glu Ala Leu Trp Leu Thr Gly Ile
   785 790 795 800
   Cys His Asn Glu Lys Asn Glu Val Met Ser Ser Gln Leu Asp Ile Asp
   805 810 815
   Asn Met Ala Gly Val Phe Tyr Met Leu Gly Ala Ala Met Ala Leu Ser
   820 825 830
   Leu Ile Thr Phe Ile Cys Glu His Leu Phe Tyr Trp Gln Phe Arg His
   835 840 845
   Cys Phe Met Gly Val Cys Ser Gly Lys Pro Gly Met Val Phe Ser Ile
   850 855 860
   Ser Arg Gly Ile Tyr Ser Cys Ile His Gly Val Ala Ile Glu Glu Arg
   865 870 875
   880
   Gln Ser Val Met Asn Ser Pro Thr Ala Thr Met Asn Asn Thr His Ser
   885 890
   895
   Asn Ile Leu Arg Leu Leu Arg Thr Ala Lys Asn Met Ala Asn Leu Ser
   900 905
   910
   Gly Val Asn Gly Ser Pro Gln Ser Ala Leu Asp Phe Ile Arg Arg Glu
   915 920 925
   Ser Ser Val Tyr Asp Ile Ser Glu His Arg Arg Ser Phe Thr His Ser
   930 935 940
   Asp Cys Lys Ser Tyr Asn Asn Pro Pro Cys Glu Glu Asn Leu Phe Ser
   945 950 955
   960
   Asp Tyr Ile Ser Glu Val Glu Arg Thr Phe Gly Asn Leu Gln Leu Lys
   965 970
   975
   Asp Ser Asn Val Tyr Gln Asp His Tyr His His His His Arg Pro His
   980 985
   990
   Ser Ile Gly Ser Ala Ser Ser Ile Asp Gly Leu Tyr Asp
   Cys Asp Asn
   995 1000
   1005
   Pro Pro Phe Thr Thr Gln Ser Arg Ser Ile Ser Lys
   Lys Pro Leu
   1010 1015 1020
   Asp Ile Gly Leu Pro Ser Ser Lys His Ser Gln Leu
   Ser Asp Leu
   1025 1030 1035
   Tyr Gly Lys Phe Ser Phe Lys Ser Asp Arg Tyr Ser
   Gly His Asp
   1040 1045 1050
   Asp Leu Ile Arg Ser Asp Val Ser Asp Ile Ser Thr
   His Thr Val
   1055 1060 1065
   Thr Tyr Gly Asn Ile Glu Gly Asn Ala Ala Lys Arg
   Arg Lys Gln
   1070 1075 1080
   Gln Tyr Lys Asp Ser Leu Lys Lys Arg Pro Ala Ser
   Ala Lys Ser
   1085 1090 1095
   Arg Arg Glu Phe Asp Glu Ile Glu Leu Ala Tyr Arg
   Arg Arg Pro
   1100 1105 1110
   Pro Arg Ser Pro Asp His Lys Arg Tyr Phe Arg Asp Lys Glu Gly
   1115 1120 1125
   Leu Arg Asp Phe Tyr Leu Asp Gln Phe Arg Thr Lys Glu Asn Ser
   1130 1135 1140
   Pro His Trp Glu His Val Asp Leu Thr Asp Ile Tyr Lys Glu Arg
   1145 1150 1155
   Ser Asp Asp Phe Lys Arg Asp Ser Ile Ser Gly Gly Gly Pro Cys
   1160 1165 1170
   Thr Asn Arg Ser His Ile Lys His Gly Thr Gly Asp Lys His Gly
   1175 1180 1185
   Val Val Ser Gly Val Pro Ala Pro Trp Glu Lys Asn Leu Thr Asn
   1190 1195 1200
   Val Glu Trp Glu Asp Arg Ser Gly Gly Asn Phe Cys Arg Ser Cys
   1205 1210 1215
   Pro Ser Lys Leu His Asn Tyr Ser Thr Thr Val Thr
   Gly Gln Asn
   1220 1225 1230
   Ser Gly Arg Gln Ala Cys Ile Arg Cys Glu Ala Cys
   Lys Lys Ala
   1235 1240 1245
   Gly Asn Leu Tyr Asp Ile Ser Glu Asp Asn Ser Leu
   Gln Glu Leu
   1250 1255 1260
   Asp Gln Pro Ala Ala Pro Val Ala Val Thr Ser Asn
   Ala Ser Thr
   1265 1270 1275
   Thr Lys Tyr Pro Gln Ser Pro Thr Asn Ser Lys Ala
   Gln Lys Lys
   1280 1285 1290
   Asn Arg Asn Lys Leu Arg Arg Gln His Ser Tyr Asp
   Thr Phe Val
   1295 1300 1305
   Asp Leu Gln Lys Glu Glu Ala Ala Leu Ala Pro Arg
   Ser Val Ser
   1310 1315 1320
   Leu Lys Asp Lys Gly Arg Phe Met Asp Gly Ser Pro
   Tyr Ala His
   1325 1330 1335
   Met Phe Glu Met Ser Ala Gly Glu Ser Thr Phe Ala
   Asn Asn Lys
   1340 1345 1350
   Ser Ser Val Pro Thr Ala Gly His His His His Asn
   Asn Pro Gly
   1355 1360 1365
   Gly Gly Tyr Met Leu Ser Lys Ser Leu Tyr Pro Asp
   Arg Val Thr
   1370 1375 1380
   Gln Asn Pro Phe Ile Pro Thr Phe Gly Asp Asp Gln
   Cys Leu Leu
   1385 1390 1395
   His Gly Ser Lys Ser Tyr Phe Phe Arg Gln Pro Thr
   Val Ala Gly
   1400 1405 1410
   Ala Ser Lys Ala Arg Pro Asp Phe Arg Ala Leu Val
   Thr Asn Lys
   1415 1420 1425
   Pro Val Val Ser Ala Leu His Gly Ala Val Pro Ala
   Arg Phe Gln
   1430 1435 1440
   Lys Asp Ile Cys Ile Gly Asn Gln Ser Asn Pro Cys
   Val Pro Asn
   1445 1450 1455
   Asn Lys Asn Pro Arg Ala Phe Asn Gly Ser Ser Asn
   Gly His Val
   1460 1465 1470
   Tyr Glu Lys Leu Ser Ser Ile Glu Ser Asp Val
   1475 1480
<210> 2
   <211> 4455
   <212> DNA
   <213> Human
<400> 2
<210> 3
   <211> 1464
   <212> PRT
   <213> Human
<400> 3
<210> 4
   <211> 4400
   <212> DNA
   <213> Human
<400> 4
<210> 5
   <211> 907
   <212> PRT
   <213> Human
<400> 5
   Arg Gly Gln Gly Gly Asp Thr Arg Ser Lys Gly Thr Arg
   Gln Pro Glu
   1 5 10
   15
   Pro Pro Gly Ala Ser Arg Asp Pro Ala Arg Ser Cys Trp
   Asp Pro Pro
   20 25
   30
   Val Asp Met Gly Gly Ala Leu Gly Pro Ala Leu Leu Leu
   Thr Ser Leu
   35 40 45
   Phe Gly Ala Trp Ala Gly Leu Gly Pro Gly Gln Gly Glu
   Gln Gly Met
   50 55 60
   Thr Val Ala Val Val Phe Ser Ser Ser Gly Pro Pro Gln
   Ala Gln Phe
   65 70 75
   80
   Arg Ala Arg Leu Thr Pro Gln Ser Phe Leu Asp Leu Pro
   Leu Glu Ile
   85 90
   95
   Gln Pro Leu Thr Val Gly Val Asn Thr Thr Asn Pro Ser
   Ser Leu Leu
   100 105
   110
   Thr Gln Ile Cys Gly Leu Leu Gly Ala Ala His Val His
   Gly Ile Val
   115 120 125
   Phe Glu Asp Asn Val Asp Thr Glu Ala Val Ala Gln Ile
   Leu Asp Phe
   130 135 140
   Ile Ser Ser Gln Thr His Val Pro Ile Leu Ser Ile Ser
   Gly Gly Ser
   145 150 155
   160
   Ala Val Val Leu Thr Pro Lys Glu Pro Gly Ser Ala Phe
   Leu Gln Leu
   165 170
   175
   Gly Val Ser Leu Glu Gln Gln Leu Gln Val Leu Phe Lys
   Val Leu Glu
   180 185
   190
   Glu Tyr Asp Trp Ser Ala Phe Ala Val Ile Thr Ser Leu
   His Pro Gly
   195 200 205
   His Ala Leu Phe Leu Glu Gly Val Arg Ala Val Ala Asp
   Ala Ser His
   210 215 220
   Val Ser Trp Arg Leu Leu Asp Val Val Thr Leu Glu Leu
   Gly Pro Gly
   225 230 235
   240
   Gly Pro Arg Ala Arg Thr Gln Arg Leu Leu Arg Gln Leu
   Asp Ala Pro
   245 250
   255
   Val Phe Val Ala Tyr Cys Ser Arg Glu Glu Ala Glu Val
   Leu Phe Ala
   260 265
   270
   Glu Ala Ala Gln Ala Gly Leu Val Gly Pro Gly His Val
   Trp Leu Val
   275 280 285
   Pro Asn Leu Ala Leu Gly Ser Thr Asp Ala Pro Pro Ala
   Thr Phe Pro
   290 295 300
   Val Gly Leu Ile Ser Val Val Thr Glu Ser Trp Arg Leu
   Ser Leu Arg
   305 310 315
   320
   Gln Lys Val Arg Asp Gly Val Ala Ile Leu Ala Leu Gly
   Ala His Ser
   325 330
   335
   Tyr Trp Arg Gln His Gly Thr Leu Pro Ala Pro Ala Gly
   Asp Cys Arg
   340 345
   350
   Val His Pro Gly Pro Val Ser Pro Ala Arg Glu Ala Phe Tyr Arg His
   355 360 365
   Leu Leu Asn Val Thr Trp Glu Gly Arg Asp Phe Ser Phe Ser Pro Gly
   370 375 380
   Gly Tyr Leu Val Gln Pro Thr Met Val Val Ile Ala Leu Asn Arg His
   385 390 395 400
   Arg Leu Trp Glu Met Val Gly Arg Trp Glu His Gly Val Leu Tyr Met
   405 410 415
   Lys Tyr Pro Val Trp Pro Arg Tyr Ser Ala Ser Leu Gln Pro Val Val
   420 425 430
   Asp Ser Arg His Leu Thr Val Ala Thr Leu Glu Glu Arg Pro Phe Val
   435 440 445
   Ile Val Glu Ser Pro Asp Pro Gly Thr Gly Gly Cys Val Pro Asn Thr
   450 455 460
   Val Pro Cys Arg Arg Gln Ser Asn His Thr Phe Ser Ser Gly Asp Val
   465 470 475 480
   Ala Pro Tyr Thr Lys Leu Cys Cys Lys Gly Phe Cys Ile Asp Ile Leu
   485 490 495
   Lys Lys Leu Ala Arg Val Val Lys Phe Ser Tyr Asp Leu Tyr Leu Val
   500 505 510
   Thr Asn Gly Lys His Gly Lys Arg Val Arg Gly Val Trp Asn Gly Met
   515 520 525
   Ile Gly Glu Val Tyr Tyr Lys Arg Ala Asp Met Ala Ile Gly Ser Leu
   530 535 540
   Thr Ile Asn Glu Glu Arg Ser Glu Ile Val Asp Phe Ser Val Pro Phe
   545 550 555 560
   Val Glu Thr Gly Ile Ser Val Met Val Ala Arg Ser Asn Gly Thr Val
   565 570 575
   Ser Pro Ser Ala Phe Leu Glu Pro Tyr Ser Pro Ala Val Trp Val Met
   580 585
   590
   Met Phe Val Met Cys Leu Thr Val Val Ala Ile Thr Val
   Phe Met Phe
   595 600 605
   Glu Tyr Phe Ser Pro Val Ser Tyr Asn Gln Asn Leu Thr
   Arg Gly Lys
   610 615 620
   Lys Ser Gly Gly Pro Ala Phe Thr Ile Gly Lys Ser Val
   Trp Leu Leu
   625 630 635
   640
   Trp Ala Leu Val Phe Asn Asn Ser Val Pro Ile Glu Asn
   Pro Arg Gly
   645 650
   655
   Thr Thr Ser Lys Ile Met Val Leu Val Trp Ala Phe Phe
   Ala Val Ile
   660 665
   670
   Phe Leu Ala Ser Tyr Thr Ala Asn Leu Ala Ala Phe Met
   Ile Gln Glu
   675 680 685
   Gln Tyr Ile Asp Thr Val Ser Gly Leu Ser Asp Lys Lys
   Phe Gln Arg
   690 695 700
   Pro Gln Asp Gln Tyr Pro Pro Phe Arg Phe Gly Thr Val
   Pro Asn Gly
   705 710 715
   720
   Ser Thr Glu Arg Asn Ile Arg Ser Asn Tyr Arg Asp Met
   His Thr His
   725 730
   735
   Met Val Lys Phe Asn Gln Arg Ser Val Glu Asp Ala Leu
   Thr Ser Leu
   740 745
   750
   Lys Met Gly Lys Leu Asp Ala Phe Ile Tyr Asp Ala Ala
   Val Leu Asn
   755 760 765
   Tyr Met Ala Gly Lys Asp Glu Gly Cys Lys Leu Val Thr
   Ile Gly Ser
   770 775 780
   Gly Lys Val Phe Ala Thr Thr Gly Tyr Gly Ile Ala Met
   Gln Lys Asp
   785 790 795
   800
   Ser His Trp Lys Arg Ala Ile Asp Leu Ala Leu Leu Gln
   Phe Leu Gly
   805 810
   815
   Asp Gly Glu Thr Gln Lys Leu Glu Thr Val Trp Leu Ser
   Gly Ile Cys
   820 825
   830
   Gln Asn Glu Lys Asn Glu Val Met Ser Ser Lys Leu Asp
   Ile Asp Asn
   835 840 845
   Met Ala Gly Val Phe Tyr Met Leu Leu Val Ala Met Gly
   Leu Ala Leu
   850 855 860
   Leu Val Phe Ala Trp Glu His Leu Val Tyr Trp Lys Leu
   Arg His Ser
   865 870 875
   880
   Val Pro Asn Ser Ser Gln Leu Asp Phe Leu Leu Ala Phe
   Ser Arg Val
   885 890
   895
   Gly Ala His Pro Ser Pro His Arg Pro Lys Phe
   900 905
<210> 6
   <211> 2724
   <212> DNA
   <213> Human
<400> 6

## Claims

1. A method of diagnosing an anti-NMDA receptor encephalitis in a subject, comprising the step of testing a biological sample obtained from the subject for an antibody to an NR subunit of an NMDA receptor, whereby a presence of said antibody in said biological sample indicates said anti-NMDA receptor encephalitis, and wherein the anti-NMDA receptor encephalitis is associated with dystonic movement, dyskinesias, or autonomic instability, thereby diagnosing said anti-NMDA receptor encephalitis in a said subject.

2. The method of claim 1, wherein the NR subunit is NR1 or NR2.

3. A method of diagnosing autoimmune encephalitis associated with a tumor in a subject, comprising the step of testing a biological sample obtained from the subject for an antibody to an NR heteromer of an NMDA receptor, whereby a presence of said antibody in said biological sample indicates an autoimmune encephalitis associated with a tumor, thereby determining a cause of said encephalitis in a subject.

4. The method of claim 3, whereby said tumor is an ovarian teratoma.

5. A method of diagnosing an occult tumor associated with an autoimmune encephalitis in a subject, comprising the step testing a body fluid obtained from the subject for an antibody to an NR heteromer of the NMDA receptor, whereby a presence of said antibody indicates a presence of an occult tumor in said subject and that said tumor is a cause of said autoimmune encephalitis.

6. The method of claim 5, whereby said autoimmune encephalitis is
(i) a paraneoplastic autoimmune encephalitis; or
(ii) an autoimmune encephalitis which comprises a limbic encephalitis; or
(iii) an autoimmune encephalitis which is associated with seizures; or
(iv) an autoimmune encephalitis which is associated with pathological symptoms.

7. The method of claim 6 (iv), whereby said pathological symptoms are psychiatric symptoms; an abnormality in cognition and behavior a movement disorder; a decreased level of consciousness; autonomic instability; hypoventilation; amnesia or a memory deficit; or a combination thereof.

8. A method of diagnosing epilepsy in a subject, comprising the step of testing a body fluid obtained from the subject for the presence of an antibody to an NR heteromer of the NMDA receptor, whereby a presence of said antibody indicates a presence of a tumor in said subject and said tumor is a cause of said epilepsy, thereby diagnosing epilepsy in a subject.

9. The method of any one of claims 3 to 8, whereby said NR heteromer is NR1 and NR2 heteromer.

10. The method of claim 9, whereby said NR heteromer
(i)is a NR2A heteromer; or
(ii) is a NR2B heteromer; or
(iii) is a NR2C heteromer.

11. The method of any one of claims 1 to 10, whereby the body fluid is cerebrospinal (CSF) fluid.

12. A method of diagnosing a tumor in a subject having an epilepsy, comprising the step of testing a body fluid obtained from the subject for an antibody to an antibody to an NR heteromer of the NMDA receptor, whereby a presence of said antibody indicates a presence of a tumor in said subject, thereby diagnosing a tumor in a subject having an epilepsy.

13. The method of claim 12, whereby the NR heteromer of the NMDA receptor is NR1, NR2 heteromer or both.

14. The method of claim 13, whereby said NR2 heteromer is a NR2B subunit, a NR2A subunit or a NR2C subunit.

## Patentansprüche

1. Verfahren zur Diagnose einer Anti-NMDA-Rezeptor-Enzephalitis in einem Probanden, umfassend den Schritt des Testens einer von dem Patienten erhaltenen biologischen Probe auf einen Antikörper gegen eine NR-Untereinheit eines NMDA-Rezeptors, wobei ein Vorhandensein des Antikörpers in der biologischen Probe die Anti-NMDA-Rezeptor-Enzephalitis anzeigt, und wobei die Anti-NMDA-Rezeptor-Enzephalitis mit dystonischer Bewegung, Dyskinesien oder autonome Instabilität verbunden ist, wodurch die Anti-NMDA-Rezeptor-Enzephalitis in dem Probanden diagnostiziert wird.

2. Verfahren nach Anspruch 1, wobei die NR-Untereinheit NR1 oder NR2 ist.

3. Verfahren zur Diagnose von Autoimmun-Enzephalitis, die mit einem Tumor verbunden ist, in einem Probanden, umfassend den Schritt des Testens einer von dem Probanden erhaltenen biologischen Probe auf einen Antikörper gegen ein NR-Heteromer eines NMDA-Rezeptors, wobei ein Vorhandensein des Antikörpers in der biologischen Probe eine Autoimmun-Enzephalitis, die mit einem Tumor verbunden ist, anzeigt, wodurch eine Ursache der Enzephalitis in dem Probanden bestimmt wird.

4. Verfahren nach Anspruch 3, wobei der Tumor ein Eierstock-Teratom ist.

5. Verfahren zur Diagnose eines okkulten Tumors, der mit einer Autoimmun-Enzephalitis verbunden ist, in einem Probanden, umfassend den Schritt des Testens einer von dem Probanden erhaltenen Körperflüssigkeit auf einen Antikörper gegen ein NR-Heteromer des NMDA-Rezeptors, wobei ein Vorhandensein des Antikörpers das Vorhandenseins eines okkulten Tumors in dem Probanden anzeigt und dass der Tumor eine Ursache der Autoimmun-Enzephalitis ist.

6. Verfahren nach Anspruch 5, wobei die Autoimmun-Enzephalitis Folgendes ist:
(i) eine paraneoplastische Autoimmun-Enzephalitis; oder
(ii) eine Autoimmun-Enzephalitis, die eine limbische Enzephalitis umfasst; oder
(iii) eine Autoimmun-Enzephalitis, die mit Krampfanfällen verbunden ist; oder
(iv) eine Autoimmun-Enzephalitis, die mit pathologischen Symptomen verbunden ist.

7. Verfahren nach Anspruch 6 (iv), wobei die pathologischen Symptome psychiatrische Symptome sind; eine Anomalie in Kognition und Verhalten; eine Bewegungsstörung; ein verminderter Bewusstseinsgrad, autonome Instabilität; Hypoventilation; Amnesie oder eine Gedächtnisschwäche; oder eine Kombination davon.

8. Verfahren zur Diagnose von Epilepsie in einem Probanden, umfassend den Schritt des Testens einer von dem Probanden erhaltenen Körperflüssigkeit auf das Vorhandensein eines Antikörpers gegen ein NR-Heteromer des NMDA-Rezeptors, wobei ein Vorhandensein des Antikörpers ein Vorhandensein eines Tumors in dem Probanden anzeigt, und der Tumor eine Ursache der Epilepsie ist, wodurch Epilepsie in einem Probanden diagnostiziert wird.

9. Verfahren nach einem der Ansprüche 3 bis 8, wobei das NR-Heteromer NR1- und NR2-Heteromer ist.

10. Verfahren nach Anspruch 9, wobei das NR-Heteromer
(i) ein NR2A-Heteromer ist; oder
(ii) ein NR2B-Heteromer ist; oder
(iii) ein NR2C-Heteromer ist.

11. Verfahren nach einem der Ansprüche 1 bis 10, wobei die Körperflüssigkeit Rückenmarksflüssigkeit (CSF, cerebrospinal fluid) ist.

12. Verfahren zur Diagnostizierung eines Tumors bei einem Probanden mit einer Epilepsie, umfassend den Schritt des Testens einer von dem Probanden erhaltenen Körperflüssigkeit auf einen Antikörper gegen einen Antikörper gegen ein NR-Heteromer des NMDA-Rezeptors, wobei ein Vorhandensein des Antikörpers das Vorhandensein eines Tumors in dem Probanden anzeigt, wodurch eine Tumor bei einem Patienten mit einer Epilepsie diagnostiziert wird.

13. Verfahren nach Anspruch 12, wobei das NR-Heteromer des NMDA-Rezeptors NR1-, NR2-Heteromer oder beides ist.

14. Verfahren nach Anspruch 13, wobei das NR2-Heteromer eine NR2B-Untereinheit, eine NR2A-Untereinheit oder eine NR2C-Untereinheit ist.

## Revendications

1. Procédé de diagnostic d'une encéphalite à anti-récepteurs NMDA chez un sujet, comprenant l'étape consistant à tester un échantillon biologique obtenu du sujet pour détecter un anticorps dirigé contre une sous-unité NR d'un récepteur NMDA, dans lequel la présence dudit anticorps dans ledit échantillon biologique indique ladite encéphalite à anti-récepteurs NMDA, et dans lequel l'encéphalite à anti-récepteurs NMDA est associée à un mouvement dystonique, à des dyskinésies ou à une instabilité du système autonome, ce qui permet le diagnostique de ladite encéphalite à anti-récepteurs NMDA chez ledit sujet.

2. Procédé selon la revendication 1, dans lequel la sous-unité NR est NR1 ou NR2.

3. Procédé de diagnostic d'une encéphalite auto-immune associée à une tumeur chez un sujet, comprenant l'étape consistant à tester un échantillon biologique obtenu dudit sujet pour déceler un anticorps dirigé contre un hétéromère NR d'un récepteur NMDA, dans lequel la présence dudit anticorps dans ledit échantillon biologique indique une encéphalite auto-immune associée à une tumeur, ce qui détermine la cause de ladite encéphalite chez un sujet.

4. Procédé selon la revendication 3, dans lequel ladite tumeur est un tératome ovarien.

5. Procédé de diagnostic d'une tumeur occulte associée à une encéphalite auto-immune chez un sujet, comprenant l'étape consistant à tester un liquide corporel obtenu du sujet pour déceler un anticorps dirigé contre un hétéromère NR du récepteur NMDA, dans lequel la présence dudit anticorps indique la présence d'une tumeur occulte chez ledit sujet et que ladite tumeur est une cause de ladite encéphalite auto-immune.

6. Procédé selon la revendication 5, dans lequel ladite encéphalite auto-immune est
(i) une encéphalite auto-immune paranéoplasique ; ou
(ii) une encéphalite auto-immune qui comprend une encéphalite limbique ; ou
(iii) une encéphalite auto-immune qui est associée à des crises d'épilepsie ; ou
(iv) une encéphalite auto-immune qui est associée à des symptômes pathologiques.

7. Procédé selon la revendication 6 (iv), dans lequel lesdits symptômes pathologiques sont des symptômes psychiatriques ; une anomalie de cognition et de comportement un trouble du mouvement ; une diminution du niveau de conscience ; une instabilité du système autonome ; une hypoventilation ; une amnésie ou un déficit de mémoire ; ou une combinaison de ceux-ci.

8. Procédé de diagnostic de l'épilepsie chez un sujet, comprenant l'étape consistant à tester un liquide corporel obtenu du sujet pour déceler la présence d'un anticorps dirigé contre un hétéromère NR du récepteur NMDA, dans lequel la présence dudit anticorps indique la présence d'une tumeur chez ledit sujet et ladite tumeur est une cause de ladite épilepsie, ce qui permet le diagnostique de l'épilepsie chez un sujet.

9. Procédé selon l'une quelconque des revendications 3 à 8, dans lequel ledit hétéromère NR est un hétéromère NR1 et de NR2.

10. Procédé selon la revendication 9, dans lequel ledit hétéromère NR
(i) est un hétéromère NR2A ; ou
(ii) est un hétéromère NR2B ; ou
(iii) est un hétéromère NR2C.

11. Procédé selon l'une quelconque des revendications 1 à 10, dans lequel le liquide corporel est du liquide céphalorachidien (LCR).

12. Procédé de diagnostic d'une tumeur chez un sujet souffrant d'épilepsie, comprenant l'étape consistant à tester un liquide corporel obtenu du sujet pour détecter un anticorps à un anticorps dirigé contre un hétéromère NR du récepteur NMDA, dans lequel la présence dudit anticorps indique la présence d'une tumeur chez ledit sujet, ce qui permet le diagnostique d'une tumeur chez un sujet souffrant d'épilepsie.

13. Procédé selon la revendication 12, dans lequel l'hétéromère NR du récepteur NMDA est un hétéromère NR1, NR2 ou l'un et l'autre.

14. Procédé selon la revendication 13, dans lequel ledit hétéromère NR2 est une sous-unité NR2B, une sous-unité NR2A ou une sous-unité NR2C.
